(19) Europäisches Patentamt

European Patent Office

Office européen des brevets

(11) **EP 0 707 826 B1**

(12) **EUROPÄISCHE PATENTSCHRIFT**

(45) Veröffentlichungstag und Bekanntmachung des Hinweises auf die Patenterteilung:
09.06.1999 Patentblatt 1999/23

(51) Int. Cl.$^6$: **A61B 5/00**

(21) Anmeldenummer: 95116266.8

(22) Anmeldetag: 04.11.1993

(54) **Verfahren und Vorrichtung zur Analyse von Glukose in einer biologischen Matrix**

Method and device for determining glucose in biologic medium

Procédé et appareil pour la détermination du glucose contenu dans un milieu biologique

(84) Benannte Vertragsstaaten:
DE ES FR GB IT

(30) Priorität: 09.11.1992 DK 136392
20.04.1993 DK 44693
21.04.1993 DK 45793
05.05.1993 DE 4314835

(43) Veröffentlichungstag der Anmeldung:
24.04.1996 Patentblatt 1996/17

(62) Dokumentnummer(n) der früheren Anmeldung(en) nach Art. 76 EPÜ:
93924502.3 / 0 659 055

(73) Patentinhaber:
Roche Diagnostics GmbH
68305 Mannheim (DE)

(72) Erfinder:
• Simonson, Jan Henning
DK-7600 Struer (DK)
• Boecker, Dirk
D-69115 Heidelberg (DE)

(74) Vertreter:
Pfeifer, Hans-Peter, Dr.,
Dr. H.-P. Pfeifer Dr. P. Jany,
Patentanwälte
Beiertheimer Allee 19
76137 Karlsruhe (DE)

(56) Entgegenhaltungen:
EP-A- 0 527 703          WO-A-93/11701
GB-A- 2 068 537          GB-A- 2 228 314
US-A- 5 057 695          US-A- 5 086 229

• PATENT ABSTRACTS OF JAPAN vol. 15 no. 421 (C-0878) ,25.Oktober 1991 & JP-A-03 173535 (MATSUSHITA ELECTRIC IND. CO LTD) 26.Juli 1991,

**Beschreibung**

Die Erfindung betrifft ein Verfahren und eine Vorrichtung zur Analyse von Glucose in einer biologischen Matrix.

Der Begriff "biologische Matrix" bezeichnet eine Körperflüssigkeit oder ein Gewebe eines lebenden Organismus. Biologische Matrices, auf die sich die Erfindung bezieht, sind optisch heterogen, d.h. sie enthalten eine Vielzahl von Streuzentren, an denen eingestrahltes Licht gestreut wird. Im Falle von biologischem Gewebe, insbesondere Hautgewebe, werden die Streuzentren von den Zellwänden und anderen in dem Gewebe enthaltenen Bestandteilen gebildet.

Körperflüssigkeiten, insbesondere Blut, sind ebenfalls optisch heterogene biologische Matrices, weil sie Partikel enthalten, an denen Licht vielfach gestreut wird. Auch Milch und andere in der Lebensmittelchemie zu untersuchende Flüssigkeiten enthalten vielfach eine hohe Konzentration von Streuzentren, beispielsweise in Form von emulgierten Fetttröpfchen.

Zur qualitativen und quantitativen analytischen Bestimmung von Komponenten solcher biologischen Matrices werden im allgemeinen Reagenzien bzw. Reagenziensysteme eingesetzt, deren Reaktion mit der jeweiligen Komponente zu einer physikalisch nachweisbaren Änderung, beispielsweise einer Änderung der Farbe der Reaktionslösung führt, die als Meßgröße gemessen werden kann. Durch Kalibration mit Standardproben bekannter Konzentration wird eine Korrelation zwischen den bei unterschiedlichen Konzentrationen gemessenen Werten der Meßgröße und der jeweiligen Konzentration bestimmt.

Diese Verfahren ermöglichen zwar Analysen mit hoher Genauigkeit und Empfindlichkeit, machen es jedoch erforderlich, eine flüssige Probe, insbesondere eine Blutprobe zur Analyse dem Körper zu entnehmen ("Invasive Analyse"). Diese Probenentnahme ist unangenehm und schmerzhaft und verursacht ein gewisses Infektionsrisiko.

Dies gilt vor allem, wenn eine Krankheit sehr häufige Analysen erforderlich macht. Das wohl wichtigste Beispiel ist der Diabetes mellitus. Um schwere Folgeerkrankungen und kritische Zustände des Patienten zu vermeiden, ist es bei dieser Krankheit erforderlich, den Glucosegehalt des Blutes sehr häufig oder sogar kontinuierlich zu bestimmen.

Es sind deshalb bereits eine Vielzahl von Verfahren und Vorrichtungen vorgeschlagen worden, um Glucose in Blut, Gewebe oder anderen biologischen Matrices in vivo und nicht-invasiv zu bestimmen.

Ein Überblick über physikochemische (reagenzienfreie) Bestimmungen von Glucose in vivo wird gegeben in: J.D. Kruse-Jarres "Physicochemical Determinations of Glucose in vivo", J. Clin. Chem. Clin. Biochem. 26 (1988), 201-208. Als nicht-invasive Verfahren werden dabei unter anderem die Kernresonanz (NMR, nuclear magnetic resonance), Elektronenspinresonanz (ESR, electron spin resonance) sowie die Infrarotspektroskopie genannt. Keines dieser Verfahren hat jedoch bis jetzt praktische Bedeutung erlangen können. Teilweise sind extrem große und aufwendige Apparaturen erforderlich, die für die Routineanalytik oder gar die Selbstkontrolle des Patienten (home monitoring) völlig ungeeignet sind.

Die Erfindung bezieht sich auf eine Teilgruppe von nichtinvasiven Analyseverfahren, bei denen Licht durch eine die biologische Matrix begrenzende Grenzfläche als Primärlicht in die biologische Matrix eingestrahlt und die Intensität des nach Wechselwirkung mit der biologischen Matrix aus dieser als Sekundärlicht austretenden Lichts gemessen wird. Eine solche Messung wird hier als "Detektionsmessung" bezeichnet. Zur Bestimmung einer Glucosekonzentration werden bei den bekannten Verfahren mehrere Detektionsmessungen bei unterschiedlichen Wellenlängen durchgeführt. Aus der bei den Detektionsmessungen ermittelten spektralen Abhängigkeit der Intensität des Sekundärlichts wird ein Meßresultat abgeleitet, das (ohne Verwendung von Reagenzien) ein Maß für die Konzentration des Analyten in der biologischen Matrix ist. Die Wellenlängen des Lichts, die für solche Verfahren diskutiert werden, liegen allgemein zwischen etwa 300 nm und mehreren tausend nm, also im Spektralbereich zwischen dem nahen UV- und infrarotem Licht. Der Begriff "Licht" darf nicht als Einschränkung auf den sichtbaren Spektralbereich des Lichtes verstanden werden.

Nahezu alle bekannten Verfahren dieser Art basieren auf den Prinzipien der Spektroskopie. Grundlage ist dabei die Wechselwirkung des eingestrahlten Primärlichtes in bestimmten spektralen Bereichen mit Vibrations- und Rotationszuständen der zu analysierenden Moleküle. Die Vibrations- und Rotations-Grundzustände der Glucose befindet sich im IR-Bereich bei Wellenlängen von mehr als 2500 nm. Diese können wegen der starken Absorption des in biologischen Matrices stets in hoher Konzentration gegenwärtigen Wassers für die nicht-invasive Analyse von Glucose nicht verwendet werden. Im Bereich des nahen Infrarot (NIR) ist die Absorption des Wassers geringer (sogenanntes "Wasser-Transmissionsfenster"). Die spektrale Analyse von Glucose in diesem Bereich basiert auf der Absorption durch Obertöne (overtones) und Kombinationsschwingungen der Vibrations- und Rotationsgrundzustände des Glucosemoleküls (vgl. vorstehend zitierter Artikel von Kruse-Jarres und EP-A-0 426 358).

Die praktische Realisierung eines nicht-invasiven Glucose-Sensors auf Basis dieser Prinzipien verursacht außerordentlich große Schwierigkeiten, die vor allem daraus resultieren, daß das Nutzsignal (die Änderung des Absorptions-Spektrums in Abhängigkeit von einer Änderung der Glucosekonzentration) sehr gering ist und diesem kleinen Nutzsignal ein großer Hintergrund von Störsignalen gegenübersteht, die insbesondere von der spektralen Absorption von Wasser und anderen stark absorbierenden Komponenten (unter anderem dem roten Blutfarbstoff Hämoglobin) resultieren. Zur Lösung dieses Problems wurden zahlreiche unterschiedliche Versuche unternommen:

- Es werden Differenz-Messungen bei unterschiedlichen Wellenlängen durchgeführt, wobei eine erste Wellenlänge so gewählt ist, daß die Glucose dort möglichst stark absorbiert, während eine zweite Wellenlänge als Referenzwellenlänge so gewählt ist, daß die Absorption bei unterschiedlichen Glucosekonzentrationen möglichst konstant ist (EP-A-0 160 768).
- In dem US-Patent 5,028,787 werden mit Hilfe von Computer-Untersuchungen Wellenlängenpaare ausgewählt, die sich in besonderem Maße für Absorptionsmessungen von Glucose eignen sollen. Als besonders gut geeignet wird das Wellenlängenpaar 945 nm und 1015 nm angesehen.
- Es werden zwei Wellenlängen so ausgewählt, daß der Extinktions-Koeffizient möglichst gleich ist. Die Intensität von zwei Strahlen mit diesen beiden Wellenlängen wird so abgeglichen, daß das detektierte Signal gleich groß ist. Änderungen der Glucosekonzentration sollen sich dabei als Änderungen des Differenzsignals zwischen beiden Wellenlängen nachweisen lassen.

Weitere Verfahren und Vorrichtungen zur nicht-invasiven Analyse von Glucose werden beschrieben in den US-Patenten 5,086,229, 4,883,953, 4,882,492 und den PCT-Anmeldungen WO 92/17765 und WO 90/07905.

Trotz dieser Bemühungen ist es bisher nicht gelungen, einen praktisch funktionsfähigen nicht-invasiven Glucose-Sensor zur Verfügung zu stellen. Realistischer ist die Möglichkeit, mit einem auf den Prinzipien der Spektralanalyse basierenden In-vivo-Sensor die Konzentration von Substanzen zu bestimmen, die um mehrere Größenordnungen stärker als Glucose absorbieren. Wichtige Beispiele sind das stark absorbierende Hämoglobin (Hb) bzw. dessen oxidierte Form $HbO_2$. Da diese Parameter Auskunft über den Oxigenierungszustand des Blutes geben, werden solche Sensoren auch als Oximeter bezeichnet. Aus der Literatur sind zahlreiche unterschiedliche Konstruktionen und Verfahren für nicht-invasive Oximeter bekannt. Verwiesen sei beispielsweise auf WO 89/01758, US 4,867,557 (entsprechend EP-A-0 286 142) , EP-A-0 353 619, EP-A-0 104 772, WO 91/17697, und die US-Patente 5,057,695, 4,223,680, 4,295,470, 4,824,242.

In der europäischen Patentschrift 0 074 428 ist ein Verfahren und eine Vorrichtung zur quantitativen Bestimmung von Glucose durch Laser-Lichtstreuung beschrieben. Dabei wird davon ausgegangen, daß die Glucosemoleküle einen durch die Lösung transmittierten Lichtstrahl streuen und daß sich daraus die Glucosekonzentration ableiten läßt. Entsprechend dieser Theorie wird die Messung darauf ausgerichtet, daß die Information über die Glucosekonzentration aus der Raumwinkelverteilung des aus einer Untersuchungsküvette oder einem untersuchten Körperteil austretenden transmittierten Lichtes erhältlich ist. Insbesondere wird die Intensität des transmittierten Lichtes in einem Winkelbereich, in dem die Änderung in Abhängigkeit von der Glucosekonzentration möglichst groß ist, gemessen und in Beziehung zu der an dem Zentralstrahl, welcher die Probe in gerader Richtung durchdringt, gemessenen Intensität gesetzt. Zur in vivo-Analyse wird ausschließlich eine Transmissionsmessung mit Laserlicht am Ohrläppchen empfohlen.

Mit ähnlichen wissenschaftlichen Überlegungen befaßt sich auch die Publikation von I.S. Chira et al.:
"Light Scattering by Blood Components after Supplying Glucose", Biomed. Technik 35 (1990), 102-106. Darin wird experimentell die Möglichkeit untersucht, die Glucose-Konzentration in Flüssigkeiten mittels Lichtstreuung zu bestimmen. Die Autoren kommen zu dem Ergebnis, daß dies weder mit statischen Lichtstreuexperimenten noch mit der Photonen-Korrelations-Spektroskopie (PCS) möglich ist.

Der Erfindung liegt die Aufgabe zugrunde, ein Verfahren für die analytische Bestimmung von Glucose in einer biologischen Matrix zur Verfügung zu stellen, welches mit einfachen Mitteln, reagenzienfrei und nicht-invasiv arbeitet und eine gute Analysegenauigkeit, zum Beispiel für die Beobachtung der Änderung der Analytkonzentration (Verlaufskontrolle) über einen ausreichenden Zeitraum, ermöglicht.

Die Aufgabe wird gelöst durch ein Verfahren nach Anspruch 1 und eine Vorrichtung nach Anspruch 14

Für die Erfindung ist kennzeichnend, daß eine für die Glucosekonzentration charakteristische Meßgröße ohne Messung mehrerer Wellenlängen dadurch bestimmt werden kann, daß zwei Detektionsmessungen (bei vorzugsweise gleicher Meßwellenlänge, aber mit unterschiedlichen Lichtwegen) stattfinden, wobei wenigstens eine der Detektionsmessungen eine ortsaufgelöste Messung von vielfach gestreutem Licht ist. Im Gegensatz zu vorbekannten spektroskopischen Verfahren (insbesondere NIR-Spektroskopie) geht es dabei nicht darum, die optische Absorption als Maß für die Glucose zu bestimmen. Die Wellenlänge wird vorzugsweise sogar in einem Spektralbereich gewählt, in dem die Absorption der Glucose verhältnismäßig gering ist.

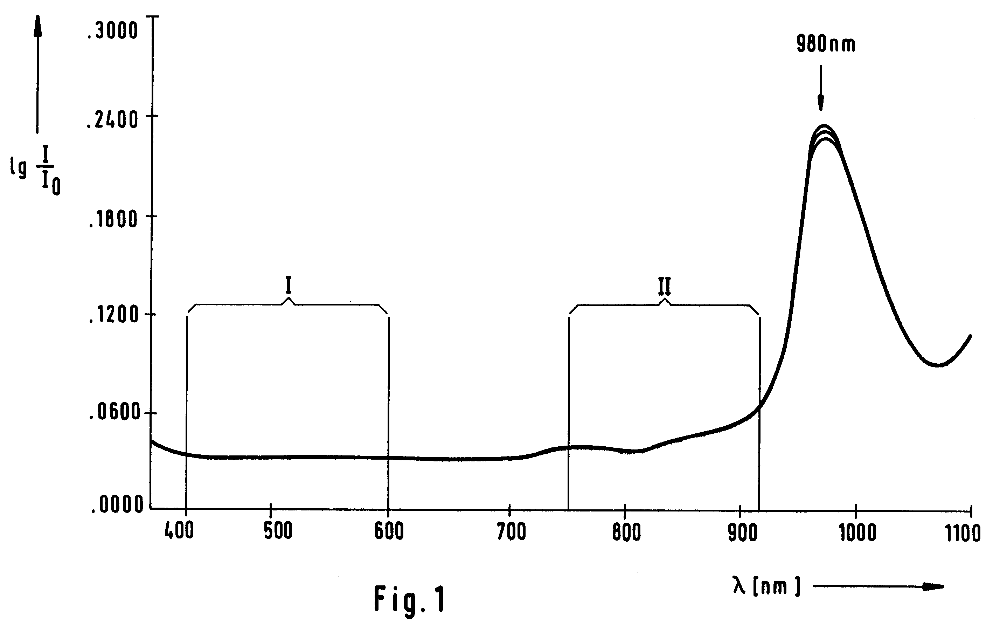

Figur 1 zeigt ein Absorptionsspektrum von Glucose in Wasser. Aufgetragen ist dabei der dekadische Logarithmus der Relation aus gemessener Intensität und eingestrahlter Intensität ($\lg I/I_0$) in Transmission für eine Küvettendicke von 1 cm und vier Glucosekonzentrationen, nämlich 0,1,5 und 10%. Man erkennt, daß sich die Spektren für diese vier Konzentrationen nur in einem kleinen Wellenlängenbereich bei etwa 980 nm geringfügig unterscheiden. Der maximale Signalunterschied zwischen dem Meßwert mit reinem Wasser und der 10%-igen Glucoselösung bei dieser Wellenlänge ist kleiner als 2 %. Bei anderen Wellenlängen ist der Unterschied noch wesentlich geringer. Dabei ist die in dem Experiment verwendete Variation der Glucosekonzentration sehr viel größer als die reale physiologische Glucosekonzentration. Bezogen auf eine realistische Glucoseänderung im physiologischen Bereich von 100 mg/dl entspricht die Änderung bei 980 nm weniger als 0,02 %. Die Änderung dI/dC des Meßsignals I in Abhängigkeit von der Glucosekonzentration C wird nachfolgend als "relativer Signalhub (relativ signal change)" bezeichnet und quantitativ in % je 100 mg/dl Änderung der Glucosekonzentration ausgedrückt.

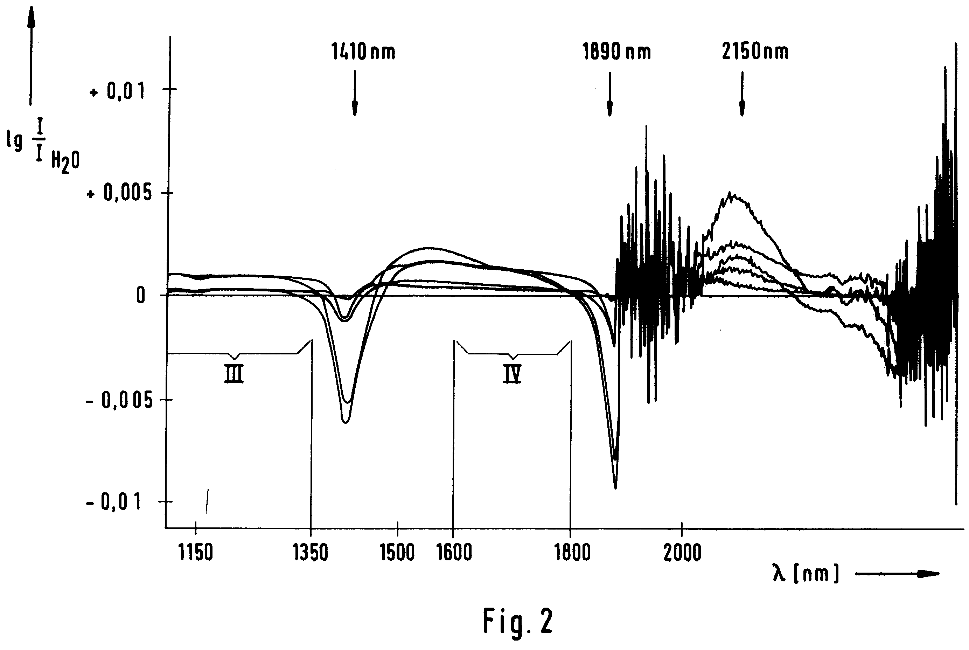

Figur 2 zeigt ein ähnliches Spektrum im anschließenden Wellenlängenbereich zwischen etwa 1100 nm und 2500 nm. Es handelt sich dabei um ein Differenzspektrum für Glucosekonzentrationen zwischen 0 und 600 mg/dl gegen reines Wasser. Negative Werte bedeuten dabei eine im Vergleich zum reinen Wasser verringerte Absorption. Dabei beträgt die maximale nutzbare Änderung der Signalintensität insgesamt weniger als 0,3 %, also im Mittel weniger als 0,05 % je 100 mg/dl Änderung der Glucosekonzentration.

Die Figuren 1 und 2 zeigen insgesamt, daß über weite Spektralbereiche die Abhängigkeit der Absorption von der Glucosekonzentration so gering ist, daß sie zur Messung in einer biologischen Matrix praktisch nicht genutzt werden kann. Als Spektralbereiche mit einer "geringen" Abhängigkeit der Absorption von der Glucosekonzentration werden die Wellenlängenbereiche angesehen, in denen der relative Signalhub dI/dC bei einer Transmissionsmessung an einer klaren Glucoselösung weniger als 0,01 % je 100 mg/dl Änderung der Glucosekonzentration beträgt.

Aufgrund der in den Figuren 1 und 2 dargestellten Meßergebnisse könnten am ehesten die Wellenlängen um etwa 980 nm, 1410 nm, 1890 nm und 2150 nm zur spektroskopischen Analyse der Glucose geeignet sein.

Im Rahmen der vorliegenden Erfindung sind dagegen die Spektralbereiche mit geringer Abhängigkeit der Absorption einer wässrigen Glucoselösung von der Glucosekonzentration vorteilhaft einsetzbar. Konkret sind sind etwa die Wellenlängen zwischen 400 nm und 940 nm, zwischen 1020 nm und 1390 nm, zwischen 1430 nm und 1880 nm, zwischen 1900 und 2050 nm sowie zwischen 2250 und 2500 nm geeignet. Besonders bevorzugt sind die in den Figuren 1 und 2 mit römischen Ziffern bezeichneten Wellenlängenbereiche, nämlich:

    I. 400 bis 600 nm.
    II. 750 bis 850 nm, vorzugsweise 780 bis 920 nm, besonders bevorzugt 780 bis 825 nm oder 850 bis 900 nm.
    III. 1050 bis 1350 nm, vorzugsweise 1200 bis 1300 nm und

    IV. 1600 bis 1800 nm, vorzugsweise 1630 bis 1770 nm, besonders bevorzugt 1630 nm bis 1670 nm oder 1730 nm bis 1770 nm.

Die Messung sollte bevorzugt näherungsweise monochromatisch sein. "Monochromatisch" ist dabei im praktischen Sinne dahingehend zu verstehen, daß der überwiegende Teil der Intensität in einem relativ engen Wellenlängenbereich emittiert und/oder detektiert wird. Die Halbwertsbreite sollte weniger als 100 nm, bevorzugt weniger als 50 nm betragen. Im Gegensatz zu den spektroskopischen Verfahren zur nicht-invasiven Glucose-Analyse können relativ breitbandige Lichtquellen (mit Halbwertsbreiten größer als 20 nm) wie beispielsweise Leuchtdioden oder andere Halbleiter-Lichtquellen ohne anschließende spektrale Selektion eingesetzt werden. Hierdurch werden die Kosten für die Apparatur erheblich verringert. Soweit hier auf "die Wellenlänge" der Lichtquelle bzw. des Primärlichts Bezug genommen wird, bezieht sich diese Aussage auf die Wellenlänge des Intensitätsmaximums.

Im Gegensatz zu den bekannten spektroskopischen Verfahren ist es ausreichend, wenn die mindestens zwei Detektionsmessungen bei jeweils nur einer Wellenlänge durchgeführt werden. Die Tatsache, daß das Meßsignal von der Wellenlänge weitgehend unabhängig ist, ermöglicht es, für die Messung solche Wellenlängenbereiche auszuwählen, bei denen die Störungen durch stark absorbierende Substanzen möglichst gering sind. In dem Bereich um 802 nm ist die gemessene Intensität näherungsweise unabhängig von dem Konzentrationsverhältnis zwischen Hb und $HbO_2$, weil diese Substanzen dort einen isosbestischen Punkt besitzen. Dies gilt auch in einem breiten isosbestischen Bereich zwischen 1200 und 1300 nm. Zusätzlich ist in diesem Bereich die Absorption von Hämoglobin und Wasser etwa gleich groß. Dadurch ergibt sich eine besonders gute Unabhängigkeit der gemessenen Intensität von dem Verhältnis von Hb, $HbO_2$ und $H_2O$.

Im Rahmen der experimentellen Erprobung der Erfindung hat sich gezeigt, daß verhältnismäßig kurze Wellenlängen, insbesondere zwischen 400 und 600 nm wegen der damit verbundenen verhältnismäßig geringen Eindringtiefe in biologischem Gewebe besonders vorteilhaft sein können. Aus experimentellen Beobachtungen bestehen Anhaltspunkte, daß dies insbesondere wegen der gleichmäßigeren Verteilung des Blutes in den obersten Hautschichten und möglicherweise auch wegen einer besseren Korrelation der Glucosekonzentration mit der Blutglucose vorteilhaft ist.

Überraschenderweise wird auf Basis der vorliegenden Erfindung in Wellenlängenbereichen, in denen die Absorption nur eine geringe Abhängigkeit von der Glucosekonzentration hat, ein relativer Signalhub dI/dC festgestellt, der sehr viel größer ist, als auf Basis der Absorption selbst in den engen Wellenlängenbereichen zu erwarten ist, in denen die Absorption der reinen Glucoselösung verhältnismäßig stark von der Glucosekonzentration abhängt. Der Wert des relativen Signalhubs ist von dem jeweiligen Meßaufbau abhängig, beträgt jedoch bei Messungen an der menschlichen Haut allgemein mehr als 0,5 % je 100 mg/dl und ist damit mindestens zehn mal so groß wie der relative Signalhub, der aufgrund der Absorptionsänderung zu erwarten wäre.

Nach dem gegenwärtigen Kenntnisstand der Erfinder läßt sich dieser Effekt wie folgt erklären.

Die Änderung der Glucosekonzentration führt zu einer Änderung des Brechungsindex der in der biologischen Matrix enthaltenen Flüssigkeit, in der die Glucose gelöst ist. Die Änderung des Brechungsindex führt zu einer Änderung der Lichtstreuung an den in der Matrix enthaltenen Streuzentren. Diese Änderung ist allerdings bei jedem einzelnen Streuprozeß extrem klein. Die Änderung des Brechungsindex pro mmol beträgt nur etwa 0,002 %. Im Rahmen der Erfindung wurde festgestellt, daß sich dieser extrem kleine Effekt zur Analyse von Glucose praktisch nutzen läßt, wenn man eine Vielzahl von Streuprozessen vieler Photonen, die einen untereinander ähnlichen Lichtweg in der biologischen Matrix durchlaufen haben, erfaßt.

Bei der Erfindung ist das Meßverfahren also so angelegt, daß idealerweise das vom Brechungsindex abhängige Streuverhalten des die Streuzentren umgebenden Raumes innerhalb der biologischen Matrix bestimmt wird. Im Rahmen der Erfindung wurde festgestellt, daß dieser Mechanismus sehr sensitiv ist, d.h. die gemessenen Signale sich bei verhältnismäßig geringen Änderungen der Glucosekonzentration stark ändern. Die erhöhte Empfindlichkeit führt im Vergleich zu bekannten in vivo-Analyse-Verfahren für Glucose auch zu einer verbesserten Selektivität, weil die Variation der erfindungsgemäß genutzten Vielfachstreuung in den wichtigsten biologischen Matrices Blut und Hautgewebe im wesentlichen nur von der Glucose-Konzentration abhängt.

Dieser erstaunliche Effekt läßt sich durch die Vielfachstreuung erklären, die im Rahmen der vorliegenden Erfindung für die Analyse der Glucose nutzbar gemacht wird. Die erfindungsgemäße Meßtechnik kann deswegen auch als "durch Vielfachstreuung verstärkte Glucosedetektion" (MSAGD; Multiple Scattering Amplified Glucose Detection) bezeichnet werden.

Aufgrund der vorliegenden Erfindung ist davon auszugehen, daß der Effekt, der der MSAGD zugrundeliegt, bei spektralanalytischen Analysen anderer Bestandteile einer optisch heterogenen biologischen Matrix eine erhebliche Störung darstellt. Es kann deshalb vorteilhaft sein, auch bei einem solchen spektralanalytischen Verfahren zur Korrektur von durch Änderungen der Glucosekonzentration verursachten Änderungen der optischen Weglänge mindestens eine ortsaufgelöste Messung von vielfach gestreutem Licht durchzuführen. Ein solches Verfahren ist ebenfalls Gegenstand der vorliegenden Erfindung.

Durch das Erfordernis der Vielfachstreuung unterscheidet sich die MSAGD grundlegend von den bisherigen Ansätzen zur nicht-invasiven Analyse. Bei der Infrarotspektroskopie, die auf der Messung der Wellenlängenabhängigkeit der Absorption basiert, wird die optische Streuung als störend empfunden. Demzufolge werden nach Möglichkeit Körperteile ausgewählt, die eine möglichst geringe Streuung des Lichtes bewirken. Beispielsweise wurde vorgeschlagen, NIR-spektroskopische Bestimmungen an der Vorkammer des Auges durchzuführen, welche eine verhältnismäßig klare und demzufolge nichtstreuende Flüssigkeit enthält (US-Patent 4,014,321).

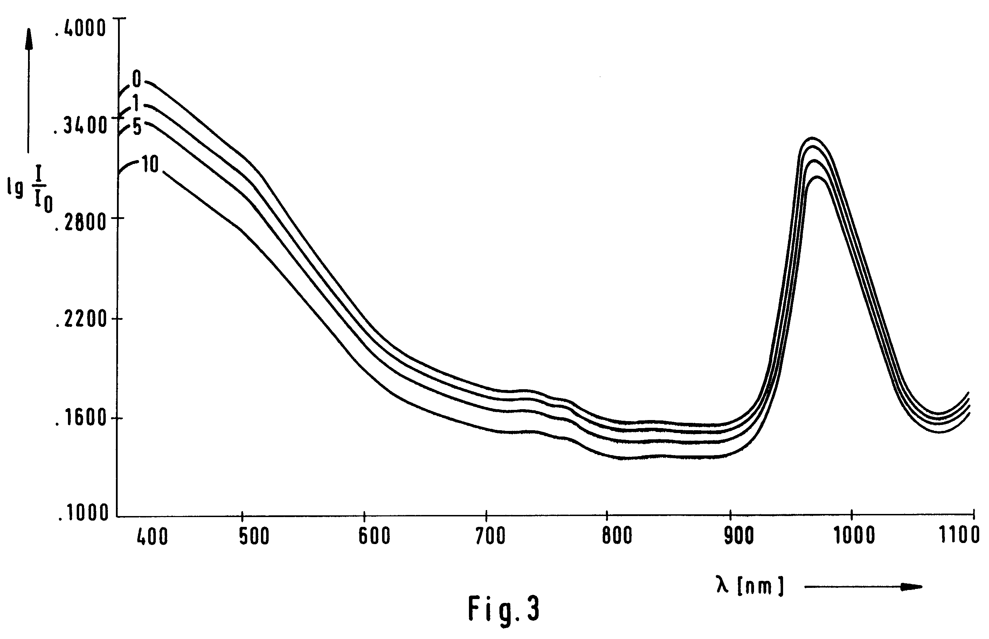

Zur Verdeutlichung sind in Figur 3 die Ergebnisse eines Laborversuchs dargestellt. Dabei wurde unter sonst gleichen Meßbedingungen der Glucoselösung, deren Spektrum in Figur 1 dargestellt ist, Milch mit einem Fettgehalt von 3,5 % in einer Konzentration von 0,1 Vol. % zugegeben. Es zeigt sich im gesamten dargestellten Spektralbereich eine starke Vergrößerung des relativen Signalhubs dI/dC, wobei dieser bei kleinen Wellenlängen besonders groß ist, insgesamt aber nur eine geringe und stetige Abhängigkeit von der Wellenlänge zeigt. Durch die Zugabe der Milch wird aus der klaren Glucoselösung eine optisch heterogene Matrix, in der dispergierte Milchtröpfchen den MSAGD-Effekt bewirken.

Wie der Vergleich der Figuren 1 und 3 zeigt, unterscheidet sich das MSAGD-Signal von Absorptionsmessungen auch dadurch, daß die Änderung der Intensität in Abhängigkeit von der Konzentration dI/dC weitgehend unabhängig von der Wellenlänge ist. Eine bestimmte Änderung der Glucosekonzentration führt demzufolge zu einer etwa gleich großen Änderung der Intensität des Sekundärlichtes, auch wenn sich die Wellenlänge des eingestrahlten Lichtes um beispielsweise 100 nm unterscheidet. Im Gegensatz dazu ist bei einer auf der Absorption der Glucose basierenden Detektionsmessung die Änderung dI/dC in einem stärker absorbierenden Wellenlängenbereich (Absorptionsbande) wesentlich höher als in einem gering absorbierenden Wellenlängenbereich. Dies wird bei spektroskopischen Messungen genutzt, um eine Wellenlänge mit starker Absorption als Meßwellenlänge und eine Wellenlänge mit schwacher Absorption als Referenzwellenlänge zu verwenden. Hierfür ist eine schmalbandige ("hochdispersive") Messung (mit einer Halbwertsbreite kleiner als 10 nm, häufig kleiner als 1 nm) in einem breiten Spektralbereich notwendig.

Bei der Erfindung ist aufgrund der geringen Abhängigkeit von der Meßwellenlänge keine schmalbandige Messung erforderlich. Es können deswegen kostengünstige Halbleiter-Lichtsender (insbesondere Leuchtdioden) verwendet werden, ohne daß zusätzliche Maßnahme zur Selektion der Wellenlänge auf der Primärseite oder auf der Sekundärseite erforderlich sind. Soweit mehrere Lichtsender mit gleicher Wellenlänge eingesetzt werden, wird eine im Rahmen der Erfindung ausreichende Gleichheit schon dann erreicht, wenn kommerziell erhältliche Halbleiter-Lichtsender mit gleicher Nominalwellenlänge verwendet werden. Deshalb kann eine erfindungsgemäße Vorrichtung besonders klein, leicht und kostengünstig aufgebaut sein. Sie eignet sich daher in besonderem Maße für die Dauerüberwachung der Glucosekonzentration eines Diabetes-Patienten.

Wesentlich für die meßtechnische Realisierung des MSAGD-Effektes an realen biologischen Matrices ist, daß die Bedingungen der ortsaufgelösten Messung von vielfach gestreutem Licht (spatially resolved measurement of multiply scattered light: "SRMMSL"; nachfolgend abgekürzt als "ortsaufgelöste Streulichtmessung" bezeichnet) erfüllt werden, d.h. es muß die "Ortsabhängigkeitsbedingung" und die "Vielfachstreuungsbedingung" eingehalten werden.

Die Ortsabhängigkeitsbedingung ist so zu verstehen, daß sich die Messung des Sekundärlichts (im Gegensatz zu der EP-A-0 074 428) nicht auf eine Strahlrichtung oder einen Winkelbereich des von dem Meßobjekt gestreuten oder des von dem Meßobjekt reflektierten Lichts bezieht, sondern auf einen definierten Teilbereich (Areal, Bezirk) einer die biologische Matrix begrenzenden Grenzfläche, der als Detektionsort bezeichnet wird. Auch die Einstrahlung des Primärlichts erfolgt in einem definierten Teilbereich einer Grenzfläche der biologischen Matrix, der als Einstrahlungsort bezeichnet wird. Eine solche Messung wird als "ortsabhängige Detektionsmessung" bezeichnet.

Die Begriffe "Einstrahlungsort" und "Detektionsort" sind (etwa im Sinne des englischen Begriffes "site") also geometrisch zu verstehen, nämlich als der Teilbereich der Grenzfläche einer biologischen Matrix, an dem Lichtstrahlen, die bei der jeweiligen Detektionsmessung für den Intensitätsmeßwert bestimmend sind, durch die Grenzfläche hindurchtreten. Als Sammelbegriff für den Eintrittsort und den Detektionsort wird nachfolgend deshalb auch die Bezeichnung "Durchtrittsort" verwendet. Soweit nachfolgend Angaben über Distanzen zwischen Durchtrittsorten gemacht werden, beziehen sich diese jeweils auf die Mitte des Einstrahlungsortes bzw. des Detektionsortes. Die Mitte wird bei einer kreisförmigen Gestaltung von dem Mittelpunkt, bei einer länglichen Gestaltung von der Mittellinie, gebildet.

Die "Vielfachstreuungsbedingung" ist so zu verstehen, daß die Durchtrittsorte (also der Einstrahlungsort und der Detektionsort) relativ zueinander so angeordnet sind, daß an Streuzentren der biologischen Matrix vielfach gestreutes Licht detektiert wird, dessen Intensität für die Konzentration der Glucose charakteristisch ist. Um dies zu gewährleisten ist folgendes zu berücksichtigen.

Die mittlere freie Weglänge von Photonen im Gewebe oder den erwähnten Körperflüssigkeiten ist von der Wellenlänge und der jeweiligen Dichte und Größe der vorhandenen Streuzentren abhängig. Typischerweise liegt sie etwa zwischen 0,01 mm und 0,1 mm. Auf dem Lichtweg in der biologischen Matrix von dem Einstrahlungsort bis zu dem Detektionsort sollten mindestens etwa 10, vorzugsweise mindestens etwa 100 Streuprozesse stattfinden. Der Lichtweg innerhalb der biologischen Matrix ist stets länger (vielfach sogar erheblich länger) als die direkte Verbindung zwischen Einstrahlungsort und Detektionsort. Als praktische Regel läßt sich jedoch angeben, daß der Abstand zwischen Einstrahlungsort und Detektionsort mindestens der zehnfachen, vorzugsweise mindestens der zwanzigfachen mittleren freien Weglänge der Photonen in der jeweiligen biologischen Matrix bei der jeweiligen Wellenlänge des Primärlichtes entsprechen sollte.

Der maximale Abstand zwischen dem Einstrahlungsort und dem Detektionsort ist ebenfalls von der mittleren freien Weglänge der Photonen abhängig und beträgt bei mindestens einer ortsaufgelösten Streulichtmessung weniger als 15 mm. Um ausschließlich an Streuzentren der biologischen Matrix vielfach gestreutes Licht zu erfassen, muß darüberhinaus auf eine sorgfältige Abschirmung des Primärlichts von dem Detektor, mit dem das Sekundärlicht gemessen wird, geachtet werden.

Die Vielfachstreuung führt dazu, daß das am Detektionsort austretende Licht weitgehend diffusen Charakter hat, d.h. seine Intensität ist weitgehend unabhängig von dem Austrittswinkel, unter dem es erfaßt wird. Wenn das Primärlicht kohärent und/oder polarisiert ist, gehen diese Eigenschaften durch die Vielfachstreuung weitgehend verloren. Aus diesem Grund muß bei der Erfindung im allgemeinen (im Gegensatz zu der EP-A-0 074 428) kein Laser als Primär-Lichtquelle verwendet werden. Auch durch den im Sekundärlicht noch erhaltenen Polarisationsgrad läßt sich testen, ob die für die Erfindung erforderliche "Vielfachstreuungsbedingung" erfüllt ist. Beispielsweise sollte der Polarisationsgrad des Sekundärlichts weniger als 10 % eines eingestrahlten polarisierten Primärlichtes betragen.

Der Einstrahlungsort und der Detektionsort können bei der ortsaufgelösten Streulichtmessung sehr unterschiedliche Dimensionen und geometrische Formgebungen haben. Wesentlich ist nur, daß durch die ortsaufgelöste Streulichtmessung eine Information über die Intensität des Sekundärlichtes in Abhängigkeit von der relativen Position des Einstrahlungsortes und des Detektionsortes (nicht etwa in Abhängigkeit vom Detektionswinkel) gewonnen wird. Mehrere solcher ortsaufgelösten Messungen von vielfach gestreutem Licht, bei denen der jeweilige Detektionsort einen unterschiedlichen Abstand vom jeweiligen Einstrahlungsort hat, ergeben somit eine Information I(D) über die funktionale Abhängigkeit der Intensität I vom Abstand D.

Im allgemeinsten Fall kann der Einstrahlungsort und vor allem der Detektionsort bei der ortsaufgelösten Streulichtmessung relativ große Abmessungen haben. Bevorzugt sind jedoch Ausführungsformen, bei denen die Durchtrittsorte in Richtung der die jeweiligen Einstrahlungs- und Detektionsorte verbindenden Abstandsgeraden (kürzeste Verbindung) eine verhältnismäßig kleine Dimension von vorzugsweise weniger als 2 mm, besonders bevorzugt weniger als 1 mm haben.

Vorzugsweise befindet sich bei der ortsaufgelösten Streulichtmessung der Detektionsort an der gleichen Grenzfläche wie der Einstrahlungsort, d.h. es wird "in Reflexion" gemessen. Soweit zwei einander gegenüberliegende Grenzflächen der biologischen Matrix zugänglich sind, kann jedoch auch "in Transmission" gemessen werden, wobei sich der Einstrahlungsort und der Detektionsort auf gegenüberliegenden Grenzflächen der biologischen Matrix befinden. Dabei dürfen die Begriffe "Transmission" und "Reflexion" im Hinblick auf den diffusen Charakter des an dem Detektionsort austretenden Lichtes natürlich nicht so verstanden werden, daß das Sekundärlicht mit einer stark dominierenden Vorzugsrichtung aus der Matrix austritt.

Bei der Erfindung werden die Intensitätsmeßwerte der mindestens zwei Detektionsmessungen verwendet, um in einem Auswerteschritt des Verfahrens mittels eines Auswertealgorithmus und einer Kalibration die Glucosekonzentration zu ermitteln ("abzuleiten"). Mindestens eine erste dieser Detektionsmessungen muß eine ortsaufgelöste Streulichtmessung sein. Bei einer zweiten Detektionsmessung kann grundsätzlich auch ein anderes Verfahren verwendet werden.

Es können auch zwei oder mehr ortsaufgelöste Streulichtmessungen durchgeführt werden, aus deren Intensitätsmeßwerten die Glucosekonzentration abgeleitet wird. Es wurde gefunden, daß aus der Abhängigkeit I(D) der Intensität I von dem Meßabstand D, die mit mindestens zwei ortsaufgelösten Streulichtmessungen bestimmt werden kann, eine besonders störungsarme und dadurch genaue Information über die Glucosekonzentration erhalten wird. Dabei werden die zwei oder mehr ortsaufgelösten Streulichtmessungen bei gleicher Wellenlänge durchgeführt. Soweit dabei mehrere Lichtsender benutzt werden, ist es ausreichend, wenn deren Wellenlänge im Rahmen üblicher Serienschwankungen von Leuchtdioden mit gleicher Nominalwellenlänge übereinstimmt.

Selbstverständlich kann auch eine größere Anzahl von ortsaufgelösten Streulichtmessungen durchgeführt und insgesamt zur Ableitung der Glucosekonzentration verwendet werden. Bei mindestens zwei dieser Messungen sollten sich die Lichtwege deutlich voneinander unterscheiden. Bei einer ortsaufgelösten Streulichtmessung ist der Begriff "Lichtweg" wegen der Vielfachstreuung in der biologischen Matrix natürlich nicht im Sinne eines geometrisch streng begrenzten Teilvolumens der biologischen Matrix (wie bei einer klassischen Transmissionsspektroskopie einer nichtstreuenden Flüssigkeit in einer Küvette) zu verstehen.

Dennoch ist es sinnvoll, den Begriff "Lichtweg" zu benutzen, wobei er beispielsweise als dasjenige Teilvolumen der biologischen Matrix verstanden werden kann, in dem ein bestimmter Prozentsatz (beispielsweise 70 %) des ausgehend von einem bestimmten Einstrahlungsort bei einem bestimmten Detektionsort eintreffenden, in der Matrix vielfach gestreuten Lichtes transportiert wird.

In der Praxis wird ein unterschiedlicher Lichtweg bei zwei ortsaufgelösten Streulichtmessungen dadurch realisiert, daß sich die Meßabstände zwischen Einstrahlungsort und Detektionsort hinreichend unterscheiden. Der durch die unterschiedlichen Lichtwege verursachte Intensitätsunterschied des Sekundärlichts (bezogen auf eine gleiche Flächenausdehnung des Detektionsortes und bei gleicher Intensität des eingestrahlten Primärlichts) sollte mindestens einen Faktor 3, vorzugsweise mindestens einen Faktor 5, besonders bevorzugt mindestens einen Faktor 10 ausmachen.

Die Tatsache, daß mehrere Detektionsmessungen bei gleicher Meßwellenlänge aber unterschiedlichem Meßabstand zwischen Einstrahlungsort und Detektionsort stattfinden, ist ein grundsätzlicher Unterschied gegenüber üblichen spektrokopischen Verfahren. Bei spektrokopischen Verfahren werden zur Gewinnung eines Wertes der gesuchten Analytkonzentration Detektionsmessungen bei mehreren Wellenlängen, jedoch völlig identischem Meßabstand, eingesetzt. Jede Änderung des Meßabstandes würde dabei eine Verfälschung des Meßergebnisses verursachen.

Besonders bevorzugt ist eine Ausführungsform, bei der vorzugsweise für zwei unterschiedliche Meßabstände jeweils mindestens zwei ortsauflösende Streulichtmessungen mit gleichem Meßabstand zwischen Einstrahlungsort und Detektionsort durchgeführt werden, bei denen sich mindestens entweder die Einstrahlungsorte oder die Detektionsorte, vorzugsweise sowohl der Einstrahlungsort als auch der Detektionsort, unterscheiden.

Eine solche Ausführungsform scheint zunächst nicht sinnvoll, weil zwei Messungen an der gleichen biologischen Matrix mit gleich langem Lichtweg zum gleichen Ergebnis führen müßten, d.h. durch die zusätzliche Messung würde keine zusätzliche Information gewonnen. Informationstechnisch ausgedrückt ist die zusätzliche Messung bei gleichem Meßabstand redundant. Im Rahmen der Erfindung wurde jedoch festgestellt, daß solche redundante Messungen vorteilhaft sind, weil sie es ermöglichen, potentielle Meßfehler durch Inhomogenitäten in der biologischen Matrix (speziell in Hautgewebe) zu erkennen und zu eliminieren.

Vorzugsweise erfolgen die mindestens zwei Detektionsmessungen gleichzeitig oder in ausreichend kurzem zeitlichem Abstand. Als ausreichend kurz ist dabei ein zeitlicher Abstand zu verstehen, bei dem zwischen den Messungen, die zur Ableitung eines Konzentrationswertes der Glucose verwendet werden, keine die Meßgenauigkeit beeinträchtigende Veränderung der biologischen Matrix stattfindet. Apparativ wird dies vorzugsweise dadurch realisiert, daß umschaltbare Einstrahlungsmittel und/oder Detektionsmittel vorgesehen sind, um ohne bewegliche Teile die Einstellung unterschiedlicher Paare von Durchtrittsorten zu ermöglichen.

Obwohl Messungen bei einer Wellenlänge ausreichen, kann es selbstverständlich sinnvoll sein, bei weiteren zusätzlichen Wellenlängen zu messen, insbesondere um Störgrößen besser eliminieren zu können. Zu diesen Störgrößen gehören unter anderem eventuelle Veränderungen der Streuzentren, sowie die Wasserabsorption und die Hämoglobin-Absorption, welche ihrerseits unmittelbar von dem Blutvolumen in der untersuchten biologischen Matrix abhängt. Hiermit im Zusammenhang steht die Tatsache, daß die Intensität des Sekundärlichts vom Blutpuls und der Körpertemperatur beeinflußt wird. Diese Einflüsse lassen sich jedoch beherrschen. Bezüglich des Blutpuls kann entweder über eine ausreichende Anzahl von Pulsperioden gemittelt oder pulssynchron gemessen werden. Die Variation der Körpertemperatur kann aufgezeichnet und zur Kompensation verwendet werden. Alternativ wird der Detektionsbereich, in dem sich die Durchtrittsfelder befinden, aktiv thermostatisiert. Da mit einer solchen Temperaturregelung ein relativ hoher Energieverbrauch verbunden ist, wird bei einer im Rahmen der Erfindung bevorzugten Vorrichtung zur in-vivo-Analyse der Meßbereich sorgfältig thermisch isoliert.

Um in dem Auswerteschritt aus den Intensitätsmeßwerten die Glucosekonzentration abzuleiten, ist ein Auswertealgorithmus und eine Kalibration erforderlich. Insoweit unterscheidet sich die Erfindung nicht wesentlich von bekannten Analyseverfahren, die ebenfalls - wie oben erläutert - eine Kalibration benötigen, um die gemessene Meßgröße (beispielsweise den Farbumschlag bei kolorimetrischen Tests) der jeweiligen Konzentration zuzuordnen.

Im einfachsten Fall enthält der Algorithmus bei der vorliegenden Erfindung eine einfache vorherbestimmte mathematische Funktion, um aus den Intensitätsmeßwerten I der Detektionsmessungen eine Zwischengröße zu ermitteln, die man als Meßresultat R bezeichnen kann. Praktisch gut bewährt hat sich eine einfache Quotientenbildung zwischen den Intensitätsmeßwerten der ersten und zweiten Detektionsmessung. Durch Kalibration mit mindestens zwei, vorzugsweise aber mehreren Standardproben bekannter Glucosekonzentration läßt sich dann in bekannter Weise das Meßresultat R mit der Konzentration C der Glucose verknüpfen.

In neuerer Zeit werden in der Analysetechnik zunehmend mathematisch anspruchsvollere Verfahren verwendet, um die Korrelation zwischen den gemessenen Meßwerten und der gesuchten Konzentration (und damit die Analysegenauigkeit) zu verbessern. Hierzu gehören exponentielle oder logarithmische Berechnungen sowie iterative Verfahren zur optimalen Beschreibung der Zuordnung. Zur Verbesserung der Analysegenauigkeit kann es darüberhinaus vorteilhaft sein, Einflußgrößen, die neben der Glucosekonzentration die gemessene Intensität beeinflussen (wie insbesondere die Temperatur am Meßort und den Puls) mit Hilfe von Korrelationsverfahren zu kompensieren. Zur Erfassung einer Vielzahl von Einflußgrößen können multilineare und nichtlineare mathematische Algorithmen bei der Auswertung von Analysemessungen eingesetzt werden. Dies ist auch bei der vorliegenden Erfindung möglich und kann vorteilhaft sein, insbesondere wenn eine Vielzahl von Detektionsmessungen mit unterschiedlichen Paarungen von Durchtrittsorten durchgeführt und der Analyse zugrundegelegt wird. In diesem Fall kann auch der Einsatz lernfähiger Systeme (neuronaler Netze) vorteilhaft sein.

Umfangreiche nähere Informationen über Auswertealgorithmen, die zur Verknüpfung der Ergebnisse von Detektionsmessungen mit der gesuchten Analytkonzentration geeignet sind, sind den einleitend genannten Druckschriften zu entnehmen.

Die Erfindung wird im folgenden anhand von in den Figuren schematisch dargestellten Ausführungsbeispielen näher erläutert; es zeigen:

| | |
|---|---|
| Fig. 1 | Ein Absorptionsspektrum von Glucose in Wasser in einem ersten Wellenlängenbereich für unterschiedliche Glucosekonzentrationen; |
| Fig. 2 | ein Differenz-Absorptionsspektrum von Glucose in Wasser gegen reines Wasser in einem zweiten Wellenlängenbereich für unterschiedliche Glucosekonzentrationen; |
| Fig. 3 | ein Spektrum entsprechend Figur 1 nach Zugabe von Milch in die Glucoselösung; |
| Fig. 4 | eine perspektivische Prinzipdarstellung einer ersten Ausführungsform der Erfindung; |
| Fig. 5 | eine perspektivische Prinzipdarstellung einer zweiten Ausführungsform der Erfindung; |
| Fig. 6 | eine perspektivische Prinzipdarstellung einer dritten Ausführungsform der Erfindung; |
| Fig. 7 | eine perspektivische Prinzipdarstellung einer vierten Ausführungsform der Erfindung; |
| Fig. 8 | eine Prinzipdarstellung eines Einstrahlungsfeldes und eines Detektionsfeldes in Aufsicht bei einer fünften Ausführungsform der Erfindung; |
| Fig. 9 bis Fig. 12 | Prinzipdarstellungen unterschiedlicher Anordnungen von Einstrahlungsorten und Detektionsorten auf einer Grenzfläche einer biologischen Matrix; |
| Fig. 13 | eine Schnittdarstellung von einer praktischen Ausführungsform eines für die Erfindung geeigne- |

ten Meßkopfes;

Fig. 14      eine Ansicht des Meßkopfes gemäß Figur 13 von der Unterseite her, die mit der biologischen Matrix kontaktiert wird;

Fig. 15      eine vergrößerte Ausschnittsdarstellung aus Figur 14;

Fig. 16      einen graphischen Vergleich der mit der Erfindung und mit einer Referenzmethode gewonnenen Analyseergebnisse;

Fig. 17      eine Seitenanschnitt-Schnittdarstellung einer Meßvorrichtung zur Analyse am Finger;

Fig. 18      eine Aufsicht-Schnittdarstellung zu der Ausführungsform von Fig. 17;

Fig. 19      eine Aufsicht auf die Meßvorrichtung bei einer Ausführungsform nach Figur 17 und 18;

Fig. 20      ein Blockschaltbild einer für die Erfindung geeigneten elektronischen Schaltung.

Fig. 21      eine Ansicht entsprechend Fig. 14 von einer alternativen Ausführungsform eines Meßkopfes;

Fig. 22      einen Schnitt durch den Lichttransmissionsbereich 34 des in Fig. 21 dargestellten Meßkopfes entlang der Schnittlinie S.

Die Figuren 1 bis 3 wurden vorstehend bereits erläutert.

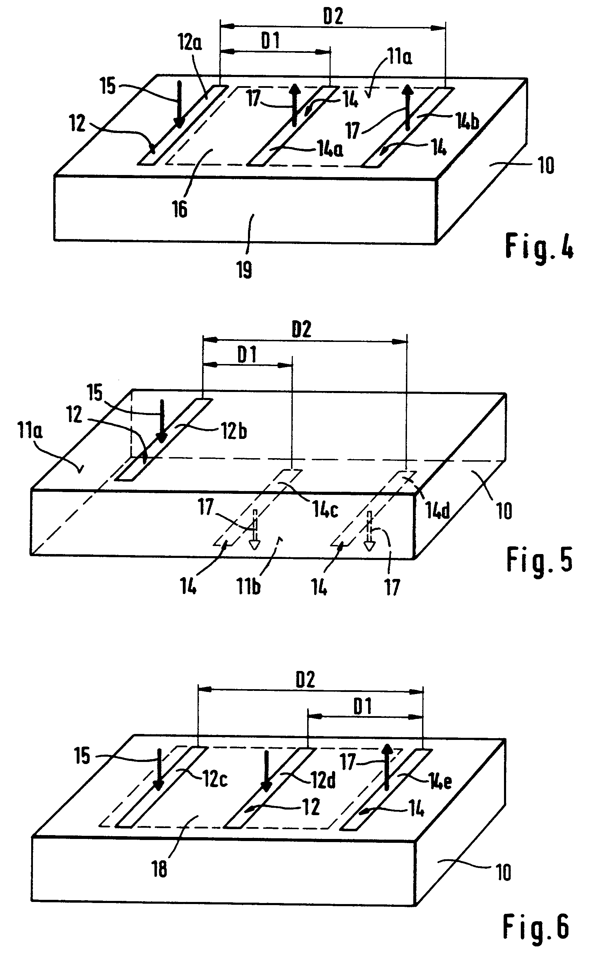

In den Figuren 4 bis 7 ist eine optisch heterogene biologische Matrix 10 symbolisch als Quader dargestellt. Sie wird durch eine obere Grenzfläche 11a und eine untere Grenzfläche 11b begrenzt. Konkret kann die biologische Matrix beispielsweise Blut sein, wobei in diesem Fall die Grenzflächen entlang den Innenwänden eines optisch transparenten Gefäßes (Küvette) verlaufen, in dem sich das Blut zur analytischen in-vitro-Untersuchung befindet. Wenn die biologische Matrix ein Gewebe ist, wird die Grenzfläche von der Oberfläche des Gewebes gebildet.

Die Figuren 4 bis 8 verdeutlichen unterschiedliche Varianten möglicher Anordnungen von einem oder mehreren Einstrahlungsorten 12 bzw. einem oder mehreren Detektionsorten 14 an einer biologischen Matrix 10 bei einer ortsaufgelösten Streulichtmessung im Sinne der Erfindung. Dabei sind die Einstrahlungsorte 12 jeweils als schmale langgestreckte Einstrahlungsfelder 12a-12f und die Detektionsorte als langgestreckte, schmale Detektionsfelder 14a-14i ausgebildet. Eine solche langgestreckte Form der Durchtrittsorte 12,14 hat sich als praktisch gut brauchbarer Kompromiß zwischen den Anforderungen einer guten räumlichen Auflösung und einer ausreichenden Intensität der eingestrahlten bzw. gemessenen Lichtes bei vertretbaren Herstellungskosten bewährt. Die Länge eines Durchtrittsfeldes sollte mindestens dreimal, bevorzugt mindestens zehnmal so groß wie seine Breite sein. Die durchschnittliche Breite der Durchtrittsfelder beträgt vorzugsweise höchstens 2 mm, besonders bevorzugt höchstens 1 mm. Soweit es nicht auf Besonderheiten der langgestreckten Form ankommt, gelten ähnliche Überlegungen jedoch auch für punkt- oder kreisförmige Durchtrittsorte.

Bei der in Figur 4 dargestellten Ausführungsform wird das Primärlicht 15 durch ein Einstrahlungsfeld 12a in die Matrix 10 eingestrahlt und das Sekundärlicht 17 detektiert, welches aus zwei in unterschiedlichen Meßabständen D1 und D2 zu dem Einstrahlungsfeld 12a verlaufende Detektionsfeldern 14a und 14b austritt.

Auch bei der in Figur 5 dargestellten Ausführungsform tritt das Primärlicht 15 durch ein Einstrahlungsfeld 12b in die biologische Matrix 10 ein und das Sekundärlicht 17 durch zwei Detektionsfelder 14c und 14d aus dieser aus, wobei die Detektionsfelder in unterschiedlichen lateralen Meßabständen D1 und D2 von dem Einstrahlungsfeld 12b angeordnet sind. Die Detektionsfelder 14c und 14d befinden sich bei dieser Ausführungsform jedoch auf der Grenzfläche 11b, die der Grenzfläche 11a, durch welche das Primärlicht 15 eingestrahlt wird, gegenüberliegt. Bei einer solchen Ausführungsform sind Eintrittsfeld und Austrittsfeld vorzugsweise so angeordnet, daß bei mindestens zwei Paaren von Durchtrittsfeldern die Oberfläche des Austrittsfeldes von keiner die Oberfläche des Einstrahlungsfeldes senkrecht kreuzenden Geraden gekreuzt wird. Mit anderen Worten sollte das Detektionsfeld nicht genau gegenüber dem Einstrahlungsfeld angeordnet, sondern stets lateral versetzt zu diesem sein.

Man kann bei der Anordnung gemäß Figur 4 von einer Messung "in Reflexion" und bei Figur 5 von einer Messung "in Transmission" sprechen, wobei diese Begriffe in dem weiter oben diskutierten Sinn zu verstehen sind.

Wenn die biologische Matrix ein Gewebe, insbesondere Hautgewebe ist, ist bei einer in vivo-Analyse in der Regel nur eine die Matrix begrenzende Grenzfläche (nämlich die Oberfläche der Haut) zugänglich. Dies gilt insbesondere für die im Rahmen der Erfindung bevorzugten Körperteile, nämlich die Fingerbeere, der Rumpf, das Nagelbett, die Skleren oder den inneren Oberarm des Menschen. In diesen Fällen ist nur die - im Rahmen der vorliegenden Erfindung ohnehin bevorzugte - Messung im Reflexionsverfahren möglich, bei der die Einstrahlung des Primärlichtes und die Detektion des Sekundärlichtes an der gleichen Grenzfläche der Matrix erfolgt. In Ausnahmefällen, beispielsweise beim Ohrläppchen, der Lippe, der Zunge oder Hautfalten (beispielsweise zwischen Daumen und Zeigefinger), stehen jedoch auch bei der in-vivo-Analyse an Hautgewebe zwei gegenüberliegende Grenzflächen 11a, 11b zur Verfügung.

Bei der in Figur 6 dargestellten Ausführungsform wird das Primärlicht durch zwei Einstrahlungsfelder 12c, 12d in die biologische Matrix 10 eingestrahlt und das dabei aus dem Detektionsfeld 14e austretende Sekundärlicht 17 detektiert. Die Einstrahlungsfelder 12d und 12c sind in unterschiedlichen Meßabständen D1 und D2 von dem Detektionsfeld 14e angeordnet, so daß auch bei dieser Ausführungsform die Möglichkeit besteht, das aus dem Detektionsfeld 14e austretende Sekundärlicht in Abhängigkeit von dem Meßabstand von dem jeweiligen Einstrahlungsfeld 12c bzw. 12d zu messen, um aus den beiden Meßwerten eine Meßgröße abzuleiten, die ein Maß für die Konzentration des Analyten in der biologischen Matrix ist.

Bei dieser Ausführungsform müssen selbstverständlich die Sekundärlichtanteile, die von dem Primärlicht der beiden unterschiedlichen Einstrahlungsfelder resultieren, voneinander getrennt werden. Dies kann zweckmäßigerweise durch zeitlich getrennte Einstrahlung erfolgen, wobei die Einstrahlung jedoch in einem ausreichend engen zeitlichen Abstand im Sinne der oben gegebenen Definition erfolgen muß. Alternativ ist es auch möglich, für die Einstrahlung in die beiden Einstrahlungsfelder 12c und 12d unterschiedlich (beispielsweise mit zwei unterschiedlichen Frequenzen) moduliertes Licht zu verwenden und die resultierenden Sekundärlichtanteile durch eine entsprechende modulationsabhängige (frequenzabhängige) Detektion, beispielsweise mit Hilfe eines Lock-in-Verstärkers zu messen.

Bei sämtlichen Ausführungsformen der Figuren 4 bis 6 beträgt der maximale Meßabstand D2 zwischen einem Einstrahlungsort und einem Detektionsort einer ortsaufgelösten Streulichtmessung 30 mm. Der kürzere Abstand D1 sollte mindestens 0,5 mm, vorzugsweise mindestens 1 mm, betragen. Mit anderen Worten sollte bei einer Ausführungsform gemäß Fig. 1 mit einem festliegenden Einstrahlungsort 12 und unterschiedlichen Detektionsorten 14 die Detektionsorte in einem (in der Figur gestrichelt eingezeichneten) Detektionsbereich 16 liegen, der den Teil der Grenzfläche 11a einschließt, der einen Abstand von mindestens 0,5 mm, bevorzugt mindestens 1 mm und höchstens 30 mm von der Mitte des Einstrahlungsortes 12 hat. Bei einer Ausführungsform gemäß Fig. 6 mit fixiertem Detektionsort 14 und unterschiedlichen Einstrahlungsorten 12 gelten die entsprechenden Werte für den dort gestrichelt eingezeichneten Einstrahlungsbereich 18.

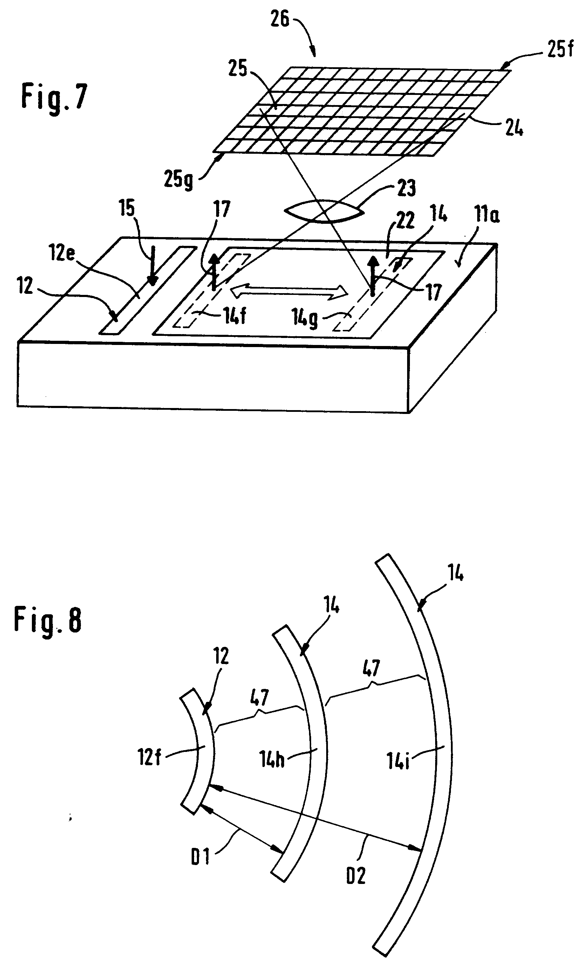

Fig. 7 zeigt eine Ausführungsform, bei der auf der Grenzfläche 11a innerhalb eines Detektionsbereiches 22 eine Vielzahl unterschiedliche Detektionsfelder 14f einstellbar sind, die in unterschiedlichen Abständen zu einem Einstrahlungsfeld 12e verlaufen. Im dargestellten Fall ist dies dadurch realisiert, daß der Detektionsbereich 22 durch ein als Linse 23 symbolisiertes optisches Abbildungssystem in eine Bildebene 24 abgebildet wird, in der sich eine zweidimensionale Anordnung 26 lichtempfindlicher Elemente befindet, welche beispielsweise als CCD's 25 (charge coupled device) realisiert sind.

Durch die optische Abbildung entspricht ein bestimmtes Detektionsfeld einem bestimmten Teilbereich der CCD-Matrix 25. Beispielsweise wird das Feld 14f auf die Reihe 25f und das Feld 14g auf die Reihe 25g der CCD-Matrix 25 abgebildet. Ein bestimmtes Detektionsfeld läßt sich folglich in einfacher Weise dadurch einstellen, daß die Intensitätsmeßsignale von denjenigen lichtempfindlichen Elementen zur Ableitung der Konzentration weiterverarbeitet werden, auf die das gewünschte Detektionsfeld abgebildet wird.

Anhand dieser Ausführungsform wird deutlich, daß die Begriffe "Einstrahlungsfeld" und "Detektionsfeld" bzw. "Einstrahlungsort" und "Detektionsort" geometrisch zu verstehen sind. Es ist nicht unbedingt erforderlich, daß irgendwelche Bauteile der Analysevorrichtung die Grenzfläche der biologischen Matrix, also beispielsweise die Oberfläche der Haut, berühren und in Kontakt zu dem Einstrahlungsort bzw. dem Detektionsort stehen. Erforderlich ist nur, daß der Einstrahlungsort und der Detektionsort für jeden Meßabstand definiert sind, d.h. das Primärlicht an einem bestimmten und begrenzten Einstrahlungsort eingestrahlt und das Sekundärlicht derartig örtlich definiert erfaßt wird, daß bekannt ist, aus welchem bestimmten abgegrenzten Teilbereich der Grenzfläche (Detektionsort) das Sekundärlicht ausgetreten ist, dessen Intensität jeweils erfaßt wird. Ein solcher Meßvorgang muß bei mindestens zwei unterschiedlichen Lichtwegen zwischen Einstrahlungsort und Detektionsort ablaufen, um aus den daraus gemessenen Intensitätsmeßwerten die für die Analyse charakteristische Meßgröße abzuleiten.

Figur 8 verdeutlicht, daß die schmale, langgestreckte Form der Felder nicht notwendigerweise (wie in den Figuren 4 bis 7) gerade sein muß. Dargestellt ist in Aufsicht auf eine Grenzfläche ein Einstrahlungsfeld 12f in Form eines Abschnitts eines Kreisringes. Zwei Detektionsfelder 14h und 14i verlaufen ebenfalls in Form von Kreisringabschnitten in unterschiedlichen Abständen zu dem Einstrahlungsfeld 12f. Die Kreisringe sind dabei konzentrisch, so daß die Detektionsfelder 14h und 14i in gleichmäßigem Abstand zu dem Einstrahlungsfeld 12f verlaufen.

Eine gute Ortsauflösung wird erreicht, wenn das Licht an einem möglichst eng begrenzten (punktförmigen) Einstrahlungsort eingestrahlt wird und der Detektionsort einen Kreis oder Kreisabschnitt um diesen Einstrahlungsort bildet. Grundsätzlich kann auch umgekehrt mit einem kreisförmigen Einstrahlungsort und einem im Zentrum dieses Kreises angeordneten punktförmigen Detektionsort gearbeitet werden, wobei hier die Signalintensität geringer ist.

Wenn - wie in den Figuren 4 bis 7 dargestellt - Einstrahlungsfelder und Detektionsfelder gerade und parallel zueinander verlaufen, ist die Ortsauflösung geringer, weil selbstverständlich an jedem Punkt eines langgestreckten Detektionsortes Lichtanteile gemessen werden, die nicht nur von dem unmittelbar gegenüberliegenden Abschnitt des Einstrahlungsfeldes, sondern auch von in Längsrichtung versetzten Abschnitten des Einstrahlungsfeldes eintreffen. Bei der praktischen Erprobung der Erfindung wurde jedoch festgestellt, daß dennoch gute Ergebnisse mit einer solchen Anordnung erreicht werden. Die Ausführungsform nach Figur 8 stellt insofern eine mittlere Lösung dar, als hier die Ortsauflösung wegen der gekrümmten konzentrischen Form besser als bei der geraden Form der Felder gemäß den Figuren 4 bis 7 ist.

Es sind auch andere gekrümmte Formen möglich, wobei jedoch allgemein die Bedingung eingehalten werden sollte, daß die Durchtrittsfelder (gemessen von Mitte zu Mitte) in gleichmäßigem Abstand D1, D2 verlaufen. Soweit sich Einstrahlungsort und Detektionsort auf der gleichen Grenzfläche der biologischen Matrix befinden, sollten der Einstrahlungsort und der Detektionsort stets von einem Streifen 47 (Figur 8) von im wesentlichen gleichmäßiger Breite getrennt sein. Die Dimension des Detektionsfeldes in Richtung der kürzesten Verbindung zu dem Einstrahlungsfeld (also in Richtung der Abstandspfeile D1,D2) sollte im Mittel weniger als 2 mm, bevorzugt weniger als 1 mm, betragen.

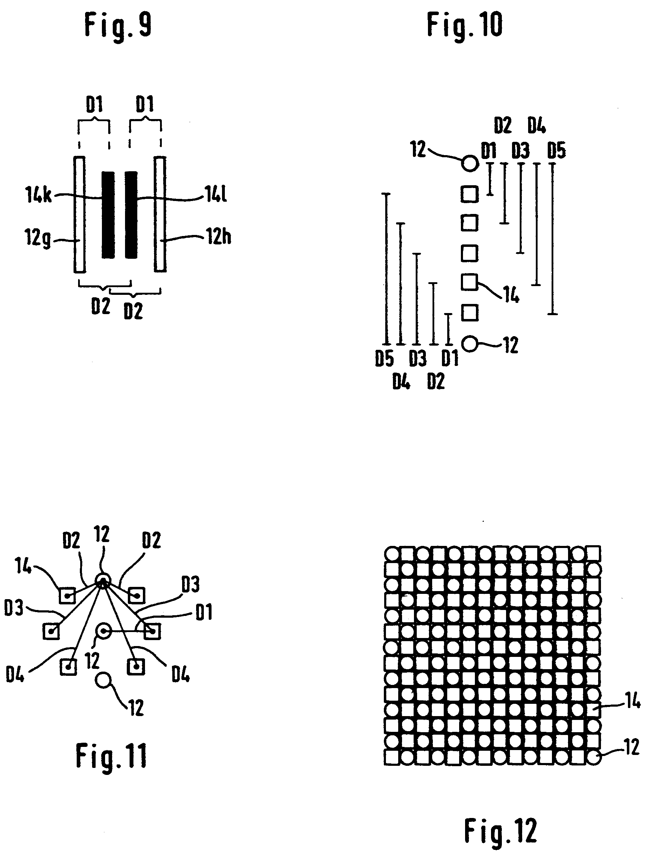

Die Figuren 9 bis 12 zeigen in einer schematisierten Darstellung (in Aufsicht auf die Grenzfläche) unterschiedliche Anordnungen, bei denen redundante Messungen möglich sind, d.h. mehrere ortsaufgelöste Streulichtmessungen mit gleichen Meßabständen und gleicher Meßwellenlänge, aber unterschiedlichen Einstrahlungs- und/oder Detektionsorten.

Bei der Ausführungsform gemäß Figur 9 sind zwei langgestreckte, rechteckige Detektionsfelder 14k,14l in gleichen Abständen zwischen zwei ebenfalls rechteckigen Einstrahlungsfeldern 12g,12h angeordnet. Sämtliche Durchtrittsfelder verlaufen parallel zueinander. Es wird deutlich, daß ein erster Meßabstand D1 sowohl durch Kombination des Einstrahlungsfeldes 12g und des Detektionsfeldes 14k als auch durch Kombination des Einstrahlungsfeldes 12h und des Detektionsfeldes 14l eingestellt werden kann. Ein zweiter größerer Meßabstand D2 ergibt sich durch Kombination der Durchtrittsfelder 12g mit 14l bzw. 12h mit 14k.

Figur 10 zeigt eine entsprechende lineare Anordnung mit zwei äußeren Einstrahlungsorten 12. Auf der Verbindungslinie zwischen den Einstrahlungsorten 12 sind in gleichmäßigen Abständen fünf Detektionsorte 14 angeordnet. Sämtliche Durchtrittsorte sind in diesem Fall punktförmig mit zweckmäßigerweise rundem Querschnitt. Die symbolische Darstellung als Quadrate und Kreise soll lediglich die Differenzierung der Einstrahlungsorte 12 und der Detektionsorte 14 erleichtern. Bei dieser Ausführungsform lassen sich fünf unterschiedliche Meßabstände D1 bis D5 jeweils in zweierlei Weise mit unterschiedlichen Einstrahlungs- und Detektionsorten einstellen, wie dies in der Figur dargestellt ist.

Bei der in Figur 11 dargestellten Ausführungsform sind drei Einstrahlungsorte 12 mit sechs Detektionsorten kombiniert. Die Einstrahlungsorte 12 sind auf einer Geraden mit gleichen Abständen angeordnet. Die Detektionsorte befinden sich im konkreten Fall auf einem Kreis um den zentralen Einstrahlungsort. Allgemein sollten sie spiegelsymmetrisch um die Gerade, auf der die Einstrahlungsorte angeordnet sind, verteilt sein. Bei dieser Anordnung ergibt sich bezüglich des zentralen Einstrahlungsortes 12 eine sechsfache Redundanz, weil jeder der Detektionsorte 14 in gleichem Abstand D1 von dem zentralen Einstrahlungsort positioniert ist. Bezüglich des oberen und unteren Einstrahlungsortes 12 ergibt sich insgesamt eine vierfache Redundanz für drei unterschiedliche Meßabstände D2,D3 und D4 (in der Figur sind der Übersichtlichkeit halber nur die Meßabstände zu dem oberen Einstrahlungsort eingezeichnet), weil jeder dieser Einstrahlungsorte jeweils mit zwei Detektionsorten kombiniert werden kann, die sich in den entsprechenden Abständen D2 bis D4 befinden. Eine solche Anordnung ermöglicht mit relativ wenigen Bauteilen eine hohe Redundanz, weil jeder Meßabstand mit mehr als zwei unterschiedlichen Kombinationen aus Einstrahlungsort 12 und Detektionsort 14 eingestellt werden kann.

Allgemein sind Anordnungen mit mindestens drei unterschiedlichen Einstrahlungsorten und mindestens drei unterschiedlichen Detektionsorten besonders bevorzugt, welche die Einstellung von mehreren unterschiedlichen Meßabständen mit jeweils mindestens drei unterschiedlichen Paarungen von Einstrahlungsort und Detektionsort ermöglichen. Eine mindestens dreifache Redundanz hat den Vorteil, daß in aller Regel erkennbar ist, wenn eine der Streulichtmessungen von den beiden anderen Streulichtmessungen abweicht. Allgemein können grundsätzlich die Einstrahlungsorte und die Detektionsorte vertauscht werden. Aus Kostengründen ist es jedoch vorteilhafter, mehr Detektionsorte als Einstrahlungsorte vorzusehen.

Bei der Ausführungsform gemäß Figur 12 sind eine Vielzahl von Einstrahlungsorten 12 und Detektionsorten 14 schachbrettartig alternierend derartig angeordnet, daß jeder Einstrahlungsort von vier Detektionsorten und jeder Detektionsort von vier Einstrahlungsorten umgeben ist. Eine solche Anordnung ermöglicht es, zahlreiche unterschiedliche Meßabstände jeweils mit einer Vielzahl unterschiedlicher Kombinationen aus Einstrahlungsorten und Detektionsorten einzustellen.

Redundante Meßanordnungen, wie sie in den Figuren 9 bis 12 dargestellt sind, ermöglichen es, potentielle Meßfehler durch Inhomogenitäten der biologischen Matrix zu erkennen und zu eliminieren. Zu diesem Zweck werden mehrere Messungen mit gleichen Meßabständen, aber unterschiedlichen Einstrahlungs- und/oder Detektionsorten durchgeführt und miteinander verglichen. Bei einer homogenen Struktur der untersuchten biologischen Matrix müßte dabei (bei gleicher Intensität des eingestrahlten Primärlichtes) die gleiche Intensität des detektierten Sekundärlichtes gemessen werden. Abweichungen lassen darauf schließen, daß in dem untersuchten Teilbereich der Hautoberfläche störende Strukturen (beispielsweise Narben, Haare oder Fibrome) vorhanden sind, die das Meßergebnis verfälschen. Dies kann auf unterschiedliche Weise korrigiert werden. Beispielsweise kann der auf die Haut aufgelegte Meßkopf in eine andere Position gebracht werden, um den untersuchten Teilbereich der Hautoberfläche ("Meßfocus") so zu ändern, daß die Inhomogenitäten außerhalb des Meßfocus liegen. Bei einer genügenden Redundanz (jedenfalls mehr als zweifach) ist es auch möglich, einzelne Meßergebnisse als Ausreißer zu erkennen und ohne Variation des Meßfocus zu eliminieren. Schließlich kommt bei einer sehr großen Anzahl von Messungen eine Mittelwertbildung in Betracht. Selbstverständlich können diese Maßnahmen auch kombiniert verwendet werden.

Wenn - wie bei der Ausführungsform gemäß Fig. 7 und Fig. 10 - eine Vielzahl von unterschiedlichen Abständen zwischen Einstrahlungsort und Detektionsort einstellbar ist, wird der Verlauf der gemessenen Intensität I in Abhängigkeit vom Meßabstand D zwischen dem jeweiligen Detektionsort und dem Einstrahlungsort erfaßt. Wenn die Detektionsorte dabei eng benachbart sind, resultiert ein praktisch kontinuierlicher Kurvenverlauf I(D), der das Profil des detektierten Sekundärlichtes in Abhängigkeit vom Abstand zwischen dem jeweiligen Einstrahlungsort und dem jeweiligen Detektionsort repräsentiert. In diesem Fall kann zur Ableitung der für die Analyse charakteristischen Meßgröße ein geeigneter, für solche Zwecke bekannter Regressionsalgorithmus verwendet werden (beispielsweise PLS).

Die Ausführungsform mit einem Detektionsbereich, in welchem eine Vielzahl unterschiedlicher Teilflächen als Detektionsorte eingestellt werden können, läßt sich selbstverständlich auch ohne das in Fig. 7 eingezeichnete optische Abbildungssystem 23 realisieren. Insbesondere ist es möglich, eine zweidimensionale Anordnung lichtempfindlicher Elemente und/oder Lichtsender unmittelbar über der Grenzfläche 11a zu positionieren, wobei mit Hilfe geeigneter Mittel, wie beispielsweise Blenden, Lichtleitern oder dergleichen, dafür Sorge getragen wird, daß jedes lichtempfindliche Element das aus einem bestimmten, abgegrenzten Teilbereich der Grenzfläche 11a austretende Sekundärlicht erfaßt. Eine solche Realisierung ist für die Ausführungsformen gemäß Fig. 10 bis 12 besonders geeignet und kann in einer Baueinheit integriert sein.

Für die Erfindung ist es vorteilhaft, wenn die Abhängigkeit der Intensität des Sekundärlichts I von dem Abstand D zwischen Einstrahlungsort und Detektionsort mit guter Ortsauflösung erfaßt wird. Deshalb sollte sowohl der Einstrahlungsort 12, als auch der Detektionsort 14 in Richtung der beide Felder verbindenden Abstandsgeraden eine kleine Abmessung von höchstens 2 mm, vorzugsweise höchstens 1 mm, haben. Vorzugsweise sind die unterschiedlichen Detektionsorte und/oder Einstrahlungsorte, durch die die unterschiedlichen Meßabstände realisiert werden, räumlich getrennt (nicht überlappend).

Eine Ausführungsform, bei der eine Vielzahl unterschiedlicher Detektionsorte mittels einer zweidimensionalen Anordnung lichtempfindlicher Elemente erfaßt werden können (wie bei Fig. 7 und Fig. 12), eröffnet eine Reihe zusätzlicher Möglichkeiten, die nachfolgend erläutert werden. Dabei sollten die Detektionsorte in dichter Folge einstellbar sein. Vorzugsweise können in mindestens einer Dimension, besonders bevorzugt in zwei Dimensionen mindestens zwei, bevorzugt mindestens vier, besonders bevorzugt mindestens acht unterschiedliche Detektionsorte pro cm eingestellt werden.

Zum einen ist es möglich, eine bestimmte Meßposition auf einer Hautoberfläche ("Meßfocus") wiederzufinden. Zum Beispiel können mit Hilfe eines Pattern Recognition Verfahrens charakteristische Strukturen der Oberfläche erkannt werden. Alternativ oder zusätzlich kann auf der Haut eine Markierung (beispielsweise mittels einer in normalem Licht unsichtbarem, im NIR-Licht jedoch kontrastierenden Tätowierung) vorgesehen sein, die von der zweidimensionalen Anordnung lichtempfindlicher Elemente erkannt und lokalisiert wird.

Zum zweiten können verschiedene Intensitätsprofile, die beispielsweise radial von einem zentralen Einstrahlungsort ausgehen, verglichen werden, um negative Einflüsse von in der Haut vorhandenen Heterogenitäten zu erkennen und gegebenenfalls zu eliminieren. Von einer Vielzahl radialer Intensitätsverteilungen, die mit Hilfe der zweidimensionalen Anordnung lichtempfindlicher Elemente erfaßt werden, werden vorzugsweise nur solche mit einem glatten Intensitätsverlauf verwendet.

Weiterhin können aufgrund der großen Datenmenge, die von einer solchen zweidimensionalen Anordnung lichtempfindlicher Elemente erzeugt wird, moderne mathematische Auswerteverfahren eingesetzt werden, die es ermöglichen, aus der Intensitätsverteilung unterschiedliche Einflußfaktoren zu differenzieren. Hierzu gehört die Möglichkeit der Trennung zwischen den Einflüssen der Gewebestruktur und der Glucosekonzentration.

Eine zweite wichtige Möglichkeit ist die Trennung der durch Absorption einerseits und Streuung andererseits verursachten Intensitätsänderung. Zu diesem Zweck müssen Intensitätsprofile mit Hilfe einer zweidimensionalen Anordnung lichtempfindlicher Elemente bei verschiedenen Wellenlängen gemessen werden. Hierzu kann vorteilhaft eine Anordnung mit vielen matrixartig dicht beieinanderangeordneten Einstrahlungs- und Detektionsorten verwendet werden, wobei in der Matrix alternierend Licht mit mindestens zwei unterschiedlichen Wellenlängen eingestrahlt bzw. detektiert wird. Die Anordnung entspricht - abgesehen von der Verwendung mehrerer Wellenlängen - im wesentlichen Fig. 12, wobei jedoch nicht notwendigerweise ein regelmäßiges Schachbrettmuster mit einer regelmäßig alternierenden Anordnung der Einstrahlungsorte und Detektionsorte verwendet werden muß.

Vorzugsweise entspricht die Anzahl der unterschiedlichen Wellenlängen der Anzahl der wesentlichen Störkomponenten plus einer weiteren Wellenlänge. Bevorzugt sind bei einer solchen Ausführungsform im Hinblick auf die drei wichtigsten Störkomponenten Hb, HbO$_2$ und H$_2$O mindestens vier unterschiedlichen Wellenlängen. Aus diesen Meßergebnissen können mittels bekannter Verfahrensweisen die Einflüsse des Streukoeffizienten und des Absorptionskoeffizienten voneinander getrennt werden. Unter Berücksichtigung der Erkenntnisse der vorliegenden Erfindung über den MSAGD-Effekt ist es mit einer solchen zweidimensionalen Mehrwellenlängen-Messung möglich, bei der Bestimmung der Glucosekonzentration mittels der vorliegenden Erfindung die Störeinflüsse der stark absorbierenden Substanzen weitgehend zu eliminieren bzw. umgekehrt beispielsweise bei einer auf der Spektralanalyse basierenden Bestimmung der Konzentration von Hb und HbO$_2$ die Störungen, die auf unterschiedliche Glucose-Konzentration zurückzuführen sind, ebenfalls weitgehend zu unterdrücken.

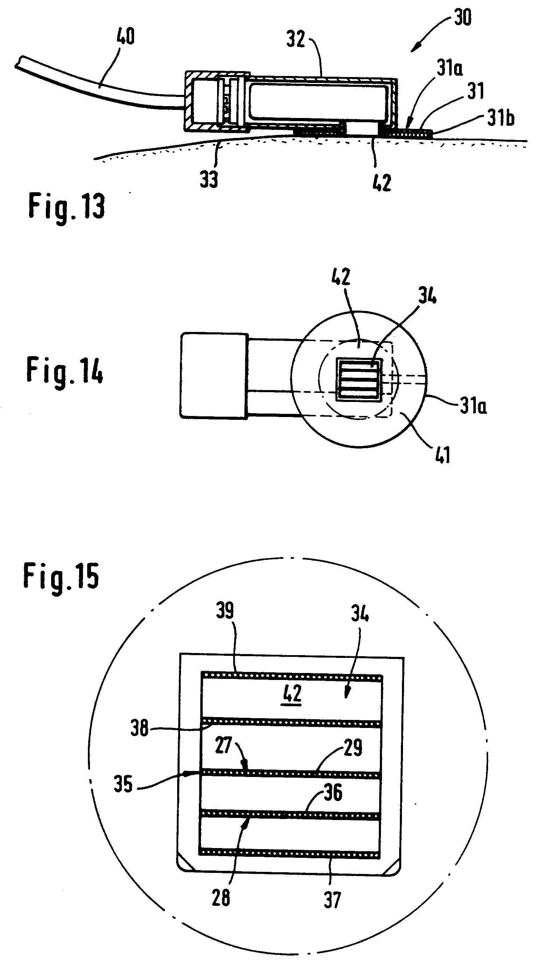

Die Figuren 13 bis 15 zeigen eine praktische Ausführungsform eines für die Erfindung geeigneten Meßkopfes 30, der speziell für die in vivo-Bestimmung von Glucose in menschlichem Gewebe geeignet ist.

Der Meßkopf 30 weist ein insgesamt etwa kreisscheibenförmig geformtes Hautkontaktteil 31 auf, welches an einem Meßkopfgehäuse 32 befestigt ist. Das Hautkontaktteil 31 wird bei der Benutzung auf die Oberfläche der Haut 33 gelegt und leicht angedrückt. In seinem Zentrum befindet sich ein quadratischer Lichttransmissionsbereich 34, der in Figur 15 vergrößert dargestellt ist. Er enthält fünf Reihen 35 bis 39 von Lichtleitfasern 29, welche im dargestellten Beispiel aus jeweils zweiunddreißig Fasern mit einem Durchmesser von jeweils 0,25 mm bestehen. Die Lichtleitfasern 29 sind in dem Lichttransmissionsbereich 34 jeweils so angeordnet, daß ihre Stirnflächen bündig in einer gemeinsamen ebenen Kontaktfläche 42 liegen und beim Auflegen des Hautkontaktteiles 31 auf die Haut 33 in unmittelbarem Kontakt mit der Haut stehen.

Die Reihe 35 von Lichtleitfasern definiert einen Einstrahlungsort. Die Lichtleitfasern 29 dieser Reihe sind zu diesem Zweck durch ein Kabel 40 mit einer nicht dargestellten Zentraleinheit verbunden, in der sich eine vorzugsweise monochromatische Lichtquelle, beispielsweise eine Leuchtdiode oder eine Laserdiode, befindet, deren Licht in die Lichtleitfasern 29 eingekoppelt wird. Die Lichtleitfasern 29 bilden zusammen mit der nicht dargestellten Lichtquelle Lichteinstrahlungsmittel 27 zum gezielten Beleuchten eines definierten Einstrahlungsortes auf einer Hautoberfläche.

Vorzugsweise wird ein Teil der Lichtleitfasern der Reihe 35 dazu verwendet, die Konstanz der Lichtquelle zu kontrollieren. In einem konkreten Experiment wurden sechzehn der zweiunddreißig Fasern zur Einstrahlung des Lichtes und die anderen sechzehn Fasern zur Kontrolle der Lichtstärke verwendet, wobei letztere getrennt von ersteren zusammengefaßt und einem lichtempfindlichen Element zugeführt werden.

Als Meßempfänger können beispielsweise Photodioden verwendet werden, die in dem Meßkopf 30 angeordnet sind. Dabei ist zweckmäßigerweise für jede Reihe 36 bis 39 von Lichtleitfasern 29, die jeweils einen möglichen Detektionsort definieren, ein gemeinsamer Meßempfänger vorgesehen. Die Lichtleitfasern dieser Reihen werden also zusammengefaßt zu jeweils einem Meßempfänger geführt, an dem das aus diesen Lichtleitfasern austretende Licht detektiert wird. Die Reihen 36 bis 39 der Lichtleitfasern 29 bilden zusammen mit dem nicht dargestellten Meßempfänger jeweils Detektionsmittel 28 zum gezielten Messen des an einem definierten Detektionsort austretenden Sekundärlichtes.

Die Stirnflächen der Lichtleitfasern der Reihen 35 bis 39 schließen jeweils bündig mit der den Lichtemissionsbereich 34 nach unten begrenzenden Hautkontaktfläche 42 ab oder stehen geringfügig daraus hervor. Dadurch wird verhindert, daß Licht entlang der Hautoberfläche unmittelbar von dem durch die Reihe 35 definierten Einstrahlungsort zu einem der Detektionsorte gelangen kann, die durch die Reihen 36 bis 39 definiert sind. Selbstverständlich sind auch innerhalb des Meßkopfes 30 die Glasfasern unterschiedlicher Reihen optisch sorgfältig voneinander getrennt, so daß keine Übertragung von Primärlicht zu den Detektionsmitteln stattfinden kann.

Der Meßkopf 30 ist insbesondere für die Verlaufskontrolle der Blutglucose von Diabetikern gedacht. Er wird zu diesem Zweck an einer geeigneten Stelle, beispielsweise an der Oberbauchdecke auf der Haut befestigt. Dies kann beispielsweise mit Hilfe eines Klebebandes erfolgen. Dabei sollte die Kontaktfläche 42 mit ausreichend festem und gleichmäßigem Druck angedrückt werden.

Um Fremdlicht abzuhalten, hat das Hautkontaktteil 31 einen Ring 31a, dessen Durchmesser wesentlich größer als der des Lichttransmissionsbereiches 34 ist. Er besteht aus einem undurchsichtigen Material und schließt mit seinem Rand 31b auf der Haut ab. Alternativ oder zusätzlich kann das Primärlicht mit einer bestimmten Frequenz moduliert sein und frequenzabhängig (beispielsweise mit Hilfe eines Lock-In-Verstärkers) schmalbandig selektiv detektiert werden, um Störlichteinflüsse zu vermindern.

Der Lichttransmissionsbereich 34 ist von einer ringförmigen Heizfläche 41 umgeben, in der eine Flächen-Widerstandsheizung angeordnet ist. Diese kann mit Hilfe eines NTC-Widerstandes und eines PD-Reglers auf eine vorbestimmte Temperatur, beispielsweise 37°, geregelt werden.

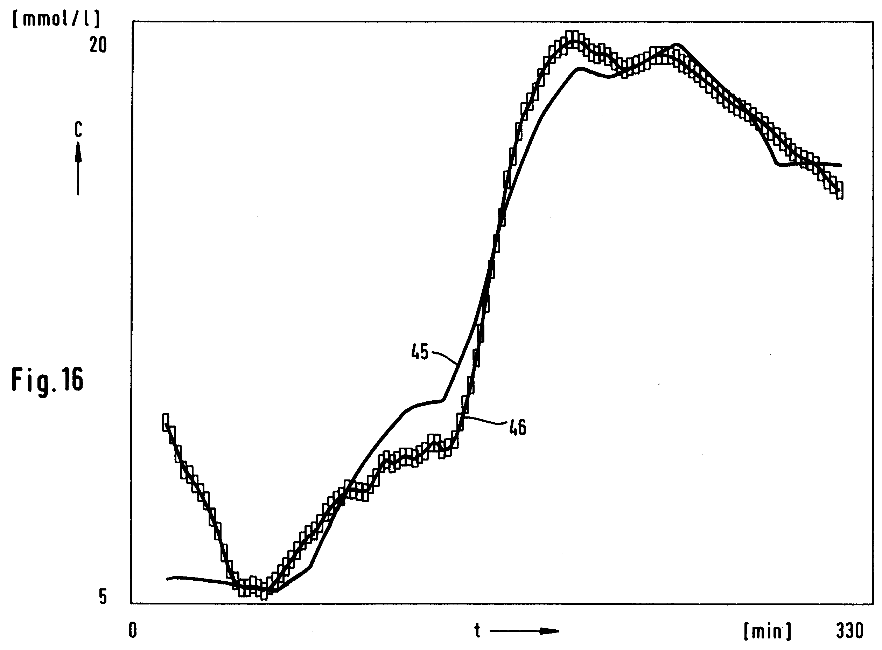

Figur 16 zeigt Analyseergebnisse, die einerseits mit einer Referenzmethode und andererseits mit einer Vorrichtung gemäß der Erfindung (Ausführungsform gemäß den Figuren 13 bis 15) erzielt wurden. Aufgetragen ist die Konzentration C in mmol/l über die Zeit t in Minuten. Die durchgezogene Linie 45 markiert die Ergebnisse einer als Referenzmethode verwendeten enzymatischen Analyse der Glucose im Blut des Probanden, während die rechteckigen Markierungen 46 Meßpunkte mit der erfindungsgemäßen Vorrichtung sind.

Bei dem erfindungsgemäßen Beispiel wurden dabei folgende Meßbedingungen eingehalten.

Es wurde ein Meßkopf gemäß den Figuren 13 bis 15 eingesetzt, wobei das Licht einer Leuchtdiode mit 1 mW Leistung und einer Wellenlänge von 805 nm durch die Lichtleitfaser-Reihe 35 in die Haut eingestrahlt und durch die Reihen 38 und 39 detektiert wurde. Der Abstand der Reihen 38 und 39 von der Reihe 35 (und folglich der Abstand der Detektionsfelder von dem Einstrahlungsfeld) betrug 3 mm bzw. 5 mm.

Zur Ableitung einer für die Konzentration charakteristischen Meßgröße R wurde der Quotient zwischen der Intensität I1 des an der Reihe 39 und der Intensität I2 des an der Reihe 38 gemessenen Lichtes gebildet. Diese Meßgröße R=I1/I2 wurde linear kalibriert gemäß der Formel

$$C = a * R + b.$$

Das in Figur 16 dargestellte Ergebnis zeigt eine ausgezeichnete Übereinstimmung der konventionell in vitro im Blut und der erfindungsgemäß in vivo im Hautgewebe gemessenen Werte über einen Zeitraum von fünfeinhalb Stunden.

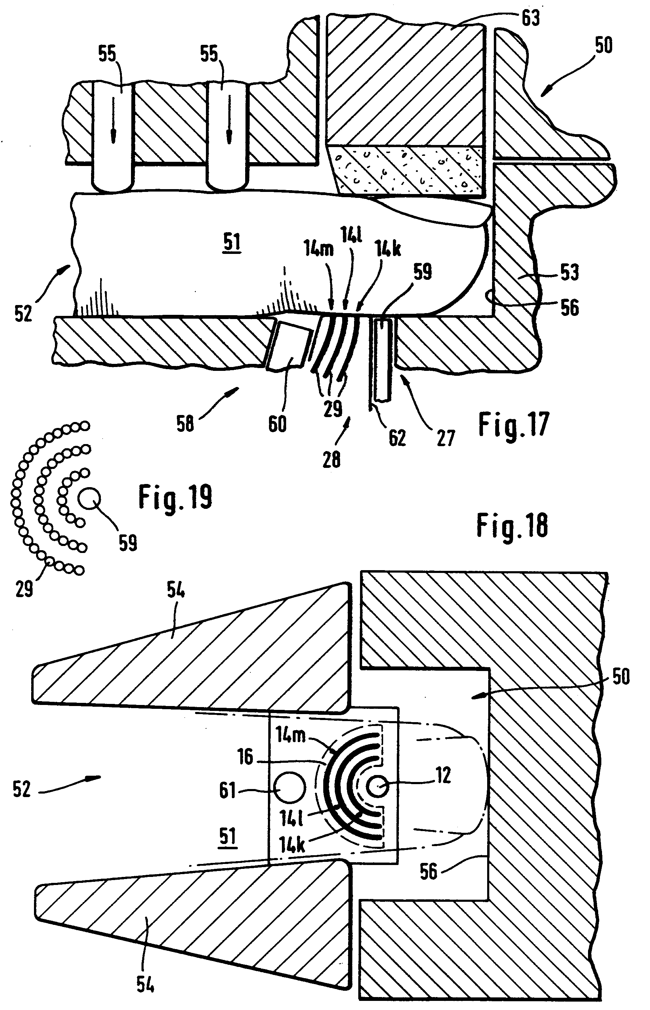

Figuren 17 und 18 zeigen eine Meßhalterung 50 zur Bestimmung der Glucosekonzentration in einem Finger 51. Der Finger 51 wird dabei in einen paßgenauen Kanal 52 eingeführt, der in einem Halterungsblock 53 ausgebildet ist. Der Halterungsblock 53 besteht aus Aluminium oder einem anderen gut wärmeleitendem Material, welches mit einer nicht dargestellten Heiz- und Thermostatisierungseinrichtung auf eine definierte Temperatur eingestellt ist, die vorzugsweise etwas über der Körper-Normaltemperatur (über 37°C) liegt.

Die den Kanal 52 seitlich begrenzenden Wandelemente 54 sind derartig verschiebbar, daß die Breite des Kanals 52 dem Finger 51 des jeweiligen Patienten angepaßt werden kann. Zur Fixierung des Fingers 51 von oben sind Fixierungselemente 55 vorgesehen, die in Richtung auf den Finger 51 gleitfähig geführt sind und mit einer nicht dargestellten Feder gegen den Finger 51 gedrückt werden.

Der Halterungsblock 53, die Wandelemente 54 und die Fixierungselemente 55 bilden zusammen mit einem den Kanal 52 in Einführrichtung begrenzenden Anschlag 56 insgesamt Fixierungsmittel, durch die der Finger 51 in einer möglichst genau reproduzierbaren Position in Bezug auf die insgesamt mit 58 bezeichnete Meßeinrichtung positioniert wird.

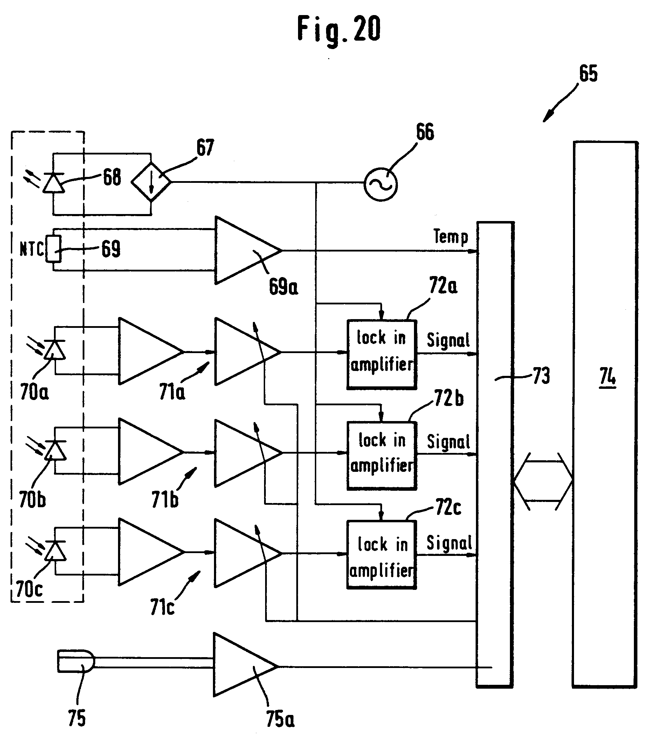

Bei der Meßeinrichtung 58 werden Lichteinstrahlungsmittel 27 durch eine nicht dargestellte Leuchtdiode und durch einen Lichtleitkanal 59 gebildet, durch die Primärlicht auf einen kreisflächenförmigen Einstrahlungsort von etwa 1 mm Durchmesser an der Unterseite der Kuppe des Fingers 51 gerichtet wird. In einem Detektionsbereich 16 sind drei Detektionsorte 14 vorgesehen, die als halbkreisförmige Detektionsfelder 14k - 14m den Einstrahlungsort 12 konzentrisch umgeben. Die Detektionsmittel 28 bestehen dabei, wie in Fig. 19 deutlicher zu erkennen ist, wiederum aus einer Reihe dicht beieinander angeordnete Lichtleitfasern 29, deren Stirnflächen den Lichtleitkanal 59 in der Hautkontaktfläche 42 halbkreisförmig umgeben und je einem Photoempfänger für das Licht von jedem der Detektionsorte 14k, 14l, 14m. Die Lichteinstrahlungsmittel 27 sind von den Detektionsmitteln 28 durch eine Trennwand 62 sorgfältig abgeschirmt.

Ein Infrarot-Temperatursensor 60 ist auf einen Temperatur-Meßort 61 gerichtet, der möglichst nah bei dem Detektionsbereich 16 liegen soll.

Bei der praktischen Erprobung der Erfindung hat es sich als wichtig erwiesen, daß der Anpreßdruck zwischen dem jeweiligen Körperteil und der Hautkontaktfläche der Meßvorrichtung ausreichend hoch und reproduzierbar ist. Bei der in den Figuren 17 und 18 dargestellten Ausführungsform wird dies mit Hilfe eines Andruckstempels 63 erreicht, der von oben auf das vorderste Glied des Fingers drückt. Als geeignet hat sich eine Andruckkraft in Höhe von etwa 300p (Pond) erwiesen.

Figur 20 zeigt beispielhaft das Blockschaltbild einer elektronischen Schaltung 65, die als Auswertemittel für eine erfindungsgemäße Meßvorrichtung geeignet ist. Von einem Oszillator 66 wird ein Spannungs-Strom-Wandler 67 angesteuert, der die Leuchtdiode 68 speist, die als Lichtquelle dient. Optional kann dabei die Temperatur der Leuchtdiode 68 durch einen NTC 69 überwacht werden, um die Konstanz der Intensität des emittierten Lichtes zu verbessern.

Die Ausgangssignale der Meßempfänger (Photodioden) 70a-70c liegen über jeweils eine Vorverstärkerschaltung 71a-71c an Lock-In-Verstärkern 72a-72c an, denen als Referenz auch das Signal des Oszillators 66 zugeleitet wird. Die Ausgangssignale der Lock-In-Verstärker 72a-72c werden in einer A/D-Wandlereinheit 73 digitalisiert und einer Mikrocomputer-Zentraleinheit 74 zugeleitet. Die Mikrocomputer-Zentraleinheit erhält darüberhinaus die Signale des NTC 69 (verstärkt durch einen Vorverstärker 69a) und eines Temperatursensors 75 (verstärkt durch einen Vorverstärker 75a) zur Messung der Temperatur in dem Detektionsbereich, welcher vorzugsweise (wie der IR-Sensor 60 der Ausführungsform nach Fig. 17) berührungslos arbeitet.

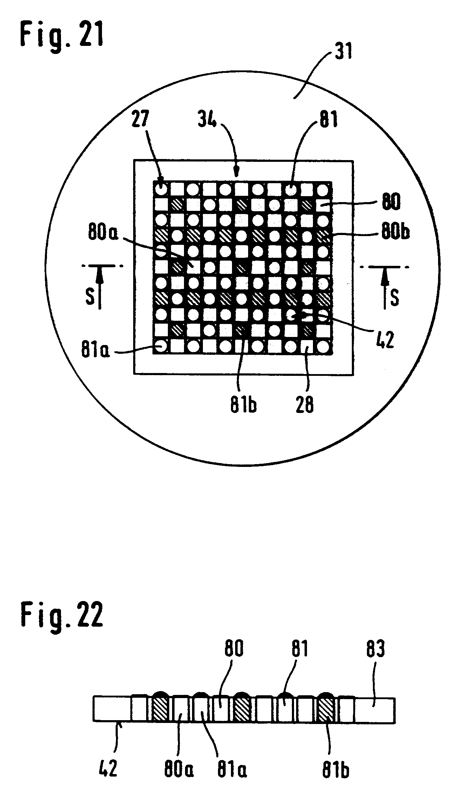

Bei der in Fig. 21 und 22 dargestellten alternativen Ausführungsform sind als Detektionsmittel 28 Halbleiter-Lichtempfänger 80 (beispielsweise Photodioden) und als Lichteinstrahlungsmittel 27 Halbleiter-Lichtsender 81 (beispielsweise Leuchtdioden) alternierend in einem matrixartigen Muster unmittelbar an der Hautkontaktfläche 42 in dem Lichttransmissionsbereich 34 des Hautkontaktfeils 31 eines Meßkopfes angeordnet. Wie in Fig. 22 deutlich zu erkennen ist, sind die Halbleiter-Lichtsender 80 und die Halbleiter-Lichtempfänger 81 in dem gleichen Bauteil 83 integriert, wobei die Anordnung ohne konstruktive Einzelheiten lediglich schematisch dargestellt ist. Die praktische Realisierung kann mit einer in der Elektronik üblichen Integrationstechnik, beispielsweise durch monolithische Integration in einem Chip oder in Hybrid-Technik erfolgen. Wesentlich ist dabei, daß sowohl die Lichtsender 81 als auch die Lichtempfänger 80 in unmittelbarem optischen Kontakt zu der Hautoberfläche stehen und gegen die Nachbarelemente abgeschirmt sind, so daß sie das Licht in einen definierten Einstrahlungsort einstrahlen bzw. an einem definierten Detektionsort detektieren.

Die dargestellte Ausführungsform erlaubt die Messung mit zwei verschiedenen Wellenlängen, beispielsweise um - wie weiter oben erläutert - Störungen durch stark absorbierende Substanzen besser eliminieren zu können. In den Figuren sind Halbleiter-Lichtsender 81a und Halbleiter-Lichtempfänger 80a für eine erste Wellenlänge weiß gezeichnet, während Halbleiter-Lichtsender 81b und Halbleiter-Lichtempfänger 80b für eine zweite Wellenlänge schraffiert dargestellt sind.

Bei sämtlichen in den Figuren dargestellten Ausführungsformen erfolgt sowohl die Einstellung unterschiedlicher Meßabstände D als auch die Einstellung unterschiedlicher Wellenlängen (soweit überhaupt erforderlich) ohne bewegliche Teile. Dies ist im Regelfall im Hinblick auf Kosten und Zuverlässigkeit vorteilhaft. Selbstverständlich sind jedoch auch Alternativen mit beweglichen Teilen im Rahmen der Erfindung möglich. So kann die Einstellung unterschiedlicher Meßabstände D dadurch erfolgen, daß entweder die Lichteinstrahlungsmittel 27 oder die Detektionsmittel 28, beispielsweise mit Hilfe eines Spindelantriebs, bewegbar sind, wobei der Verlauf I(D) der Intensität I in Abhängigkeit von dem Meßabstand D durch schrittweises Verstellen dieses Antriebs bestimmt wird.

In besonderen Fällen kann es auch zweckmäßig sein, beispielsweise zum Erfassen von Störgrößen, Mittel zum Einstrahlen und/oder Detektieren eines schmalbandigen Wellenlängenbereiches vorzusehen. Zu diesem Zweck kann auf der Primärseite oder auf der Sekundärseite ein Gittermonochromator vorgesehen sind.

**Patentansprüche**

1. Verfahren zur Bestimmung der Konzentration von Glucose in einer biologischen Matrix (10), umfassend

    mindestens zwei Detektionsmessungen, bei denen jeweils Licht durch eine die biologische Matrix (10) begrenzende Grenzfläche (11a) als Primärlicht (15) in die biologische Matrix (10) eingestrahlt wird, das Licht in der biologischen Matrix (10) entlang einem Lichtweg propagiert und eine aus der biologischen Matrix (10) durch eine diese begrenzende Grenzfläche (11a, 11b) als Sekundärlicht (17) austretende Lichtintensität gemessen wird, und

einen Auswerteschritt, bei dem aus den Intensitätsmeßwerten der Detektionsmessungen mittels eines Auswertealgorithmus und einer Kalibration die Glucosekonzentration abgeleitet wird,

**dadurch gekennzeichnet**, daß mindestens eine Detektionsmessung eine ortsaufgelöste Messung von vielfach gestreutem Licht ist, bei der das Primärlicht (15) an einem definierten Einstrahlungsort (12) in die biologische Matrix eingestrahlt wird, die Intensität des an einem definierten Detektionsort (14) in einem Abstand von höchstens 15 mm von der Mitte des Einstrahlungsortes (12) aus der biologischen Matrix austretenden Sekundärlichts (17) gemessen wird und der Detektionsort (14) relativ zu dem Einstrahlungsort (12) so angeordnet ist, daß an Streuzentren (19) der biologischen Matrix (10) vielfach gestreutes Licht detektiert wird.

2. Verfahren zur spektralanalytischen Analyse eines Analyten in einer biologischen Matrix, umfassend

    mindestens zwei Detektionsmessungen zur Messung der spektralen Abhängigkeit der Absorption des Analyten bei mindestens zwei unterschiedlichen Wellenlängen, bei denen jeweils Licht durch eine die biologische Matrix (10) begrenzende Grenzfläche (11a) als Primärlicht (15) in die biologische Matrix (10) eingestrahlt wird, das Licht in der biologischen Matrix (10) entlang einem Lichtweg propagiert und eine aus der biologischen Matrix (10) durch eine diese begrenzende Grenzfläche (11a, 11b) als Sekundärlicht (17) austretende Lichtintensität gemessen wird, und

einen Auswerteschritt, bei dem aus den Intensitätsmeßwerten der Detektionsmessungen mittels eines Auswertealgorithmus und einer Kalibration die Analytkonzentration abgeleitet wird,

**dadurch gekennzeichnet**, daß zur Korrektur von durch Änderung der Glucosekonzentration verursachten Änderung der optischen Weglänge mindestens eine ortsaufgelöste Messung von vielfach gestreutem Licht durchgeführt wird, bei der das Primärlicht (15) an einem definierten Einstrahlungsort (12) in die biologische Matrix eingestrahlt wird, die Intensität des an einem definierten Detektionsort (14) aus der biologischen Matrix austretenden Sekundärlichts (17) gemessen wird und der Detektionsort (14) in einem Abstand von höchstens 15 mm von der Mitte des Einstrahlungsortes (12) relativ zu dem Einstrahlungsort (12) so angeordnet ist, daß an Streuzentren (19) der biologischen Matrix (10) vielfach gestreutes Licht detektiert wird.

3. Verfahren nach einem der vorhergehenden Ansprüche, **dadurch gekennzeichnet**, daß bei der mindestens einen ortsaufgelösten Streulichtmessung die Abmessung des Einstrahlungsortes (12) in Richtung der ihn mit dem Detektionsort (14) verbindenden Abstandsgeraden weniger als 2 mm, bevorzugt weniger als 1 mm beträgt.

4. Verfahren nach einem der vorhergehenden Ansprüche, **dadurch gekennzeichnet**, daß bei der mindestens einen ortsaufgelösten Streulichtmessung der relative Signalhub dI/dC mehr als 0,5 % je 100 mg/dl Änderungen der Glucosekonzentration beträgt.

5. Verfahren nach einem der vorhergehenden Ansprüche, **dadurch gekennzeichnet**, daß bei der mindestens einen ortsaufgelösten Streulichtmessung die Wellenlänge des Primärlichtes in einem Wellenlängenbereich mit einer geringen Abhängigkeit der optischen Absorption von der Glucosekonzentration liegt.

6. Verfahren nach einem der vorhergehenden Ansprüche, **dadurch gekennzeichnet**, daß die Wellenlänge des Primärlichts zwischen 400 nm und 2500 nm liegt.

7. Verfahren nach einem der vorhergehenden Ansprüche, **dadurch gekennzeichnet**, daß zur Elimination von Störungen Detektionsmessungen bei mehreren unterschiedlichen Wellenlängen durchgeführt werden.

8. Verfahren nach einem der vorhergehenden Ansprüche, **dadurch gekennzeichnet**, daß mehrere ortsaufgelöste Messungen von vielfach gestreutem Licht bei gleicher Wellenlänge des Primärlichtes durchgeführt werden.

9. Verfahren nach einem der vorhergehenden Ansprüche, **dadurch gekennzeichnet**, daß in einem Detektionsbereich eine Vielzahl von unterschiedlichen Teilflächen als Detektionsorte (14) eingestellt werden, um die Intensität des an einer Vielzahl von Detektionsorten (14) austretenden Sekundärlichts (17) zu messen und dadurch eine Vielzahl von ortsaufgelösten Messungen von vielfach gestreutem Licht durchzuführen.

10. Verfahren nach Anspruch 9, **dadurch gekennzeichnet**, daß die Dichte der in dem Detektionsbereich eingestellten unterschiedlichen Detektionsorte in mindestens einer Dimension mindestens zwei Detektionsorte, bevorzugt mindestens vier Detektionsorte pro Zentimeter beträgt.

11. Verfahren nach Anspruch 9 oder 10, **dadurch gekennzeichnet**, daß die in dem Detektionsbereich (22) aus der biologischen Matrix (10) austretende Lichtintensität von einer zweidimensionalen Anordnung (26) lichtempfindlicher Elemente (25) ortsaufgelöst erfaßt wird.

12. Verfahren nach einem der vorhergehenden Ansprüche, **dadurch gekennzeichnet**, daß in dem Auswerteschritt die Einflüsse der Absorption und der Streuung der biologischen Matrix durch Auswertung der Intensitätsverteilung des Sekundärlichts (17) als Funktion des Abstandes des Detektionsortes (14) von dem Einstrahlungsort (12) getrennt werden.

13. Verfahren nach einem der vorhergehenden Ansprüche, **dadurch gekennzeichnet**, daß die biologische Matrix (10) ein biologisches Gewebe ist.

14. Vorrichtung zur Bestimmung der Konzentration von Glucose in einer biologischen Matrix zur Durchführung des Verfahrens nach einem der vorhergehenden Ansprüche, mit

einem zum Anlegen an eine Grenzfläche der biologischen Matrix vorgesehenen Lichttransmissionsbereich (34),
Einstrahlungsmitteln (27) zum Einstrahlen von Licht in die biologische Matrix (10) durch eine diese begrenzende Grenzfläche (11a,11b),
Detektionsmitteln (28) zum Messen der Intensität von aus der biologischen Matrix (10) durch eine diese begrenzende Grenzfläche (11a,11b) austretendem Licht und
Auswertemitteln zum Umwandeln der gemessenen Intensität in ein der Glucosekonzentration entsprechendes Signal,
**dadurch gekennzeichnet**, daß
die Einstrahlungsmittel (27) zum gezielten Beleuchten eines definierten Einstrahlungsortes (12) ausgebildet sind und die Detektionsmittel (28) zum gezielten Messen des an einem definierten Detektionsort (14) austretenden Sekundärlichts ausgebildet sind, wobei der Detektionsort (14) in einem Abstand von höchstens 15 mm von der Mitte des Einstrahlungsortes (12) relativ zu dem Einstrahlungsort (12) so angeordnet ist, daß an Streuzentren (19) der biologischen Matrix (10) vielfach gestreutes Licht detektiert wird.

**15.** Vorrichtung nach Anspruch 14, **dadurch gekennzeichnet**, daß mindestens zwei Lichteinstrahlungsmittel (27) mit im wesentlichen gleicher Wellenlänge vorgesehen sind, die zum gezielten Beleuchten unterschiedlicher, räumlich nicht überlappender Einstrahlungsorte ausgebildet sind.

**16.** Vorrichtung nach Anspruch 14 oder 15, **dadurch gekennzeichnet**, daß mindestens zwei Detektionsmittel (28) vorgesehen sind, die zum gezielten Messen des an zwei unterschiedlichen, räumlich nicht überlappenden Detektionsorten aus der biologischen Matrix austretenden Sekundärlichts ausgebildet sind.

**17.** Vorrichtung nach einem der Ansprüche 14 bis 16, **dadurch gekennzeichnet**, daß die Detektionsmittel eine zweidimensionale Anordnung (26) lichtempfindlicher Elemente (25,80) einschließen.

**18.** Vorrichtung nach Anspruch 17, **dadurch gekennzeichnet**, daß eine Vielzahl von Lichtempfängern (80) in einem Lichttransmissionsbereich (34) in einem Bauteil (83) integriert sind.

**Claims**

**1.** Method for the determination of the glucose concentration in a biological matrix (10), comprising

at least two detection measurements, in each of which light is irradiated as primary light (15) into the biological matrix (10) through a boundary surface (11a) bounding the biological matrix (10), the light is propagated along a light path within the biological matrix (10) and the intensity of the light is measured as it emerges as secondary light (17) from the biological matrix (10) through a boundary surface (11a, 11b) thereof, and
an evaluation step, in which the glucose concentration is derived from the measured intensities of the detection measurements with the aid of an evaluation algorithm and a calibration, characterized in that
at least one detection measurement is a spatially resolved measurement of multiply scattered light, in which the primary light (15) is irradiated into the biological matrix at a defined irradiation site (12), the intensity of the secondary light (17) emerging from the biological matrix at a defined detection site (14) at a distance of at least 15 mm from the centre of the irradiation site (12) is measured, and the detection site (14) is located relative to the irradiation site (12) so that light which was multiply scattered on scattering centres (19) in the biological matrix (10) is detected.

**2.** Method for the spectral analytical determination of an analyte in a biological matrix, comprising

at least two detection measurements for measurement of the spectral dependence of the absorption of the analyte at at least two different wavelengths, in which in each instance light is irradiated as primary light (15) into the biological matrix (10) through a boundary surface (11a) bounding the biological matrix (10), the light is propagated along a light path within the biological matrix (10) and the intensity of the light is measured as it emerges as secondary light (17) from the biological matrix (10) through a boundary surface (11a, 11b) thereof, and
an evaluation step, in which the analyte concentration is derived from the measured intensities of the detection measurements by means of an evaluation algorithm and a calibration, characterized in that
in order to correct for changes in the optical path length caused by a change in the glucose concentration, at least one spatially resolved measurement of multiply scattered light is carried out, in which the primary light (15) is irradiated into the biological matrix at a defined irradiation site (12), the intensity of the secondary light (17) emerging from the biological matrix at a defined detection site (14) is measured, and the detection site (14) is located at a distance of at least 15 mm from the centre of the irradiation site (12) in such a manner relative to the irradiation site (12) that light which was multiply scattered on scattering centres (19) in the biological matrix (10) is detected.

**3.** Method according to any one of the preceding claims, characterized in that in the at least one spatially resolved measurement of multiply scattered light the dimension of the irradiation site (12) in the direction defined by the shortest connection to the detection site (14) is less than 2 mm, preferably less than 1 mm.

**4.** Method according to any one of the preceding claims, characterized in that in the at least one spatially resolved measurement of multiply scattered light the relative signal change dI/dC per 100 mg/dl change in the glucose concentration is more than 0.5 %.

**5.** Method according to any one of the preceding claims, characterized in that in the at least one spatially resolved measurement of multiply scattered light the wavelength of the primary light is in a region in which there is a small dependence of the optical absorption on the glucose concentration.

6. Method according to any one of the preceding claims, characterized in that the wavelength of the primary light is between 400 nm and 2500 nm.

7. Method according to any one of the preceding claims, characterized in that a plurality of detection measurements is performed at different wavelengths in order to eliminate interferences.

8. Method according to any one of the preceding claims, characterized in that a plurality of spatially resolved measurements of multiply scattered light are made with the same wavelength of the primary light.

9. Method according to any one of the preceding claims, characterized in that in a detection area a plurality of different sub-areas are provided as detection sites (14) for measurement of the intensity of the secondary light (17) emerging at the plurality of detection sites (14), thereby to perform a plurality of spatially resolved measurements of multiply scattered light.

10. Method according to claims 9, characterized in that the density of the different detection sites provided in the detection area in at least one dimension is at least two detection sites, preferably at least four detection sites per centimetre.

11. Method according to any one of claims 9 to 10, characterized in that the intensity of the light emerging from the biological matrix (10) in the detection area (22) is measured in a spatially resolved manner by a two-dimensional array (26) of photosensitive elements (25).

12. Method according to any one of the preceding claims, characterized in that in the evaluation step the contributions of absorption and scattering in the biological matrix are separated by evaluating the intensity profile of the secondary light (17) as a function of the distance of the detection site (14) from the irradiation site (12).

13. Method according to any one of the preceding claims, characterized in that the biological matrix is a biological tissue.

14. Apparatus for the determination of the glucose concentration in a biological matrix, for carrying out the method according to any one of the preceding claims, with

a light-transmission area (34) adapted for application to a boundary surface of the biological matrix,
irradiation means (27) for irradiating light into the biological matrix (10) through one of the boundary surfaces (11a, 11b) bounding the biological matrix,
detection means (28) for measuring the intensity of the light emerging from the biological matrix (10) through a boundary surface (11a, 11b) bounding the biological matrix (10) and evaluation means for converting the measured intensity into a signal corresponding to the glucose concentration,
characterized in that
the irradiation means (27) are designed to provide spatially limited illumination of a defined irradiation site (12) and the detection means (28) are designed for the spatially limited measurement of the secondary light emerging at a defined detection site (14), where the detection site (14) is located at a distance of at least 15 mm from the centre of the irradiation site (12) in such a manner relative to the irradiation site (12) that light multiply scattered at scattering centres (19) in the biological matrix (10) is detected.

15. Apparatus according to claim 14, characterized in that at least two irradiation means (27) with essentially identical wavelength are provided, which are designed for the spatially limited illumination of different, spatially non-overlapping irradiation sites.

16. Apparatus according to any one of claims 14 or 15, characterized in that at least two detection means (28) are provided, which are designed for the spatially limited measurement of the secondary light emerging from the biological matrix at two different, spatially non-overlapping detection sites.

17. Apparatus according to any one of claims 14 to 16, characterized in that the detection means comprise a two-dimensional array (26) of photosensitive elements (25, 80).

18. Device according to claim 17, characterized in that a plurality of light receivers (80) in the light transmission area (34) are integrated in one component (83).

**Revendications**

1. Procédé pour la détermination de la concentration en glucose dans une matrice biologique (10), comprenant

au moins deux mesures de détection, selon chacune desquelles on irradie une lumière, en tant que lumière primaire (15), dans la matrice biologique (10) au travers d'une surface frontière (11a) délimitant la matrice biologique, la lumière se propage dans la matrice biologique (10) le long d'un chemin optique et on mesure une intensité lumineuse, en tant que lumière secondaire (17), provenant de la matrice biologique (10) au travers d'une de ces surfaces frontières (11a, 11b) délimitantes, et

une étape d'évaluation, au cours de laquelle, à partir des valeurs mesurées de l'intensité des mesures de détection, on détermine la concentration en glucose au moyen d'un algorithme d'évaluation et d'un étalonnage, caractérisé en ce que,

au moins une mesure de détection est une mesure localement résolue de lumière plusieurs fois dispersée, dans laquelle la lumière primaire (15) est irradiée à un lieu d'incidence défini (12) dans la matrice biologique, l'intensité de la lumière secondaire (17) sortant de la matrice biologique est mesurée en un lieu de détection défini (14) à une distance de 15 mm au plus du milieu du lieu d'incidence (12) et le lieu de détection (14) est disposé par rapport au lieu défini d'incidence (12) de façon à détecter de la lumière plusieurs fois dispersée sur des centres dispersifs (19) de la matrice biologique (10).

2. Procédé d'analyse au moyen d'une analyse spectrale d'un analyte dans une matrice biologique, comprenant

au moins deux mesures de détection pour mesurer la dépendance spectrale de l'absorption de l'analyte à au moins deux longueurs d'onde différentes, dans lesquelles à chaque fois une lumière est irradiée en tant que lumière primaire (15), dans la matrice biologique (10), à travers une surface frontière (11a) de la matrice biologique (10), la lumière se propage dans la matrice biologique (10) le long d'un chemin optique et une intensité lumineuse émergeant de la matrice biologique (10) au travers d'une de ces surfaces (11a, 11b) délimitant celle-ci est détectée en tant que lumière secondaire (17), et

une étape d'évaluation, dans laquelle la concentration en analyte est déterminée à partir des valeurs mesurées de l'intensité des mesures de détection à l'aide d'un algorithme d'évaluation et d'un étalonnage, caractérisé en ce que

au moins une mesure localement résolue de lumière plusieurs fois dispersée est effectuée pour la correction de la modification de chemin optique due à la modification de la concentration en glucose, dans laquelle la lumière primaire (15) est irradiée dans la matrice biologique à un lieu d'incidence défini (12), l'intensité de la lumière secondaire (17) émergeant de la matrice biologique est mesurée à un lieu de détection défini (14), et le lieu de détection (14) est disposé à une distance de 15 mm au plus du milieu du lieu d'incidence (12) par rapport au lieu d'incidence (12) afin de détecter de la lumière plusieurs fois dispersée sur des centres dispersifs (19) de la matrice biologique (10).

3. Procédé selon l'une quelconque des revendications précédentes, caractérisé en ce qu'au cours d'au moins une des mesures localement résolues de lumière plusieurs fois dispersée, la dimension du lieu d'incidence (12) dans la direction définie par la plus courte distance au lieu de détection (14) est inférieure à 2 mm, de préférence inférieure à 1 mm.

4. Procédé selon l'une quelconque des revendications précédentes, caractérisé en ce qu'au cours d'au moins une des mesures localement résolues de lumière plusieurs fois dispersée, l'excursion relative du signal dI/dC s'élève à plus de 0,5% pour chaque variation de 100 mg/dl de la concentration en glucose.

5. Procédé selon l'une quelconque des revendications 1 à 9, caractérisé en ce qu'au cours d'au moins une des mesures localement résolues de lumière plusieurs fois dispersée, la longueur d'onde de la lumière primaire est située dans un domaine de longueurs d'onde présentant une plus faible dépendance de l'absorption optique en fonction de la concentration en glucose.

6. Procédé selon l'une quelconque des revendications précédentes, caractérisé en ce que la longueur d'onde de la lumière primaire est comprise entre 400 nm et 2500 nm.

7. Procédé selon l'une quelconque des revendications précédentes, caractérisé en ce que des mesures de détection à plusieurs longueurs d'onde différentes sont effectuées afin d'éliminer les perturbations.

8. Procédé selon l'une quelconque des revendications précédentes, caractérisé en ce que plusieurs mesures localement résolues de lumière plusieurs fois dispersée sont effectuées à la même longueur d'onde de la lumière primaire.

9. Procédé selon l'une quelconque des revendications précédentes, caractérisé en ce que, dans une zone de détection, une pluralité de surfaces partielles différentes sont prévues en tant que lieux de détection (14), afin de mesurer l'intensité de la lumière secondaire (17) émergeant d'une pluralité de lieux de détection (14) et ainsi d'effectuer une pluralité de mesures localement résolues de lumière plusieurs fois diffusée.

10. Procédé selon la revendication 9, caractérisé en ce que la densité des différents lieux de détection prévus dans la zone de détection est, selon au moins une dimension, au moins de deux lieux de détection par centimètre, de préférence au moins de quatre lieux de détection par centimètre.

11. Procédé selon la revendication 9 ou 10, caractérisé en ce que l'intensité de la lumière émergeant de la matrice biologique (10) est acquise de façon localement résolue dans la zone de détection (22) par une matrice bidimensionnelle (26) d'éléments photosensibles (25).

12. Procédé selon l'une quelconque des revendications précédentes, caractérisé en ce que dans l'étape d'évaluation, les influences de l'absorption et de la dispersion dans la matrice biologique sont séparées au moyen de l'évaluation de la distribution de l'intensité de la lumière secondaire (17) en tant que fonction de la distance séparant le lieu de détection (14) du lieu d'incidence (12).

13. Procédé selon l'une quelconque des revendications précédentes, caractérisé en ce que la matrice biologique (10) est un tissu biologique.

14. Dispositif pour la détermination de la concentration en glucose dans une matrice biologique, mettant en oeuvre le procédé selon l'une quelconque des revendications précédentes, avec

une zone de transmission de la lumière (34) prévue pour être disposée à une surface frontière de la matrice biologique,

des moyens d'irradiation (27) afin d'irradier une lumière dans la matrice biologique (10) au travers d'une de ces surfaces (11a, 11b) délimitant celle-ci,

des moyens de détection (28) afin de mesurer l'intensité de la lumière émergeant de la matrice biologique (10) au travers d'une de ces surfaces frontières (11a, 11b) délimitant celle-ci, et

des moyens d'évaluation afin de transformer l'intensité mesurée en un signal correspondant à la concentration en glucose,
caractérisé en ce que

les moyens d'irradiation (27) sont conçus dans le but d'éclairer de manière dirigée un lieu d'incidence (12) défini et les moyens de détection (28) sont conçus dans le but de mesurer la lumière secondaire en un lieu de détection (14) défini, dans lequel le lieu de détection (14) est disposé à une distance de 15 mm au plus du milieu du lieu d'incidence (12) par rapport au lieu d'incidence (12) afin de détecter la lumière plusieurs fois diffusée sur les centres de diffusion (19) de la matrice biologique (10).

15. Dispositif selon la revendication 14, caractérisé en ce qu'on prévoit au moins deux moyens d'irradiation lumineuse (27) avec essentiellement les mêmes longueurs d'onde qui sont réalisés dans le but d'irradier des lieux d'incidence différents qui ne se chevauchent pas spatialement.

16. Dispositif selon la revendication 14 ou 15, caractérisé en ce qu'on prévoit au moins deux moyens de détection (28) qui sont réalisés dans le but de mesurer la lumière secondaire émergeant de la matrice biologique en deux lieux de détection différents qui ne se chevauchent pas spatialement.

17. Dispositif selon l'une quelconque des revendications 14 à 16, caractérisé en ce que les moyens de détection (28) incluent une matrice bidimensionnelle (26) d'éléments photosensibles (25, 80).

18. Dispositif selon la revendication 17, caractérisé en ce qu'une pluralité de photorécepteurs (80) dans un domaine de transmission de la lumière (34) sont intégrés dans une pièce de construction (83).

Fig.1

EP 0 707 826 B1

Fig. 2

Fig. 3

EP 0 707 826 B1

Fig.4

Fig.5

Fig.6

Fig.7

Fig.8

# Fig.9

# Fig.10

# Fig.11

# Fig.12

Fig.13

Fig.14

Fig.15

Fig.16

Fig.17

Fig.19

Fig.18

# Fig. 20

# Fig. 21

# Fig. 22